(19) Europäisches Patentamt
European Patent Office
Office européen des brevets

(11) **EP 3 753 569 A1**

(12) **EUROPEAN PATENT APPLICATION**
published in accordance with Art. 153(4) EPC

(43) Date of publication:
**23.12.2020 Bulletin 2020/52**

(21) Application number: **19753814.3**

(22) Date of filing: **14.02.2019**

(51) Int Cl.:
*A61K 38/17* (2006.01)    *A23L 2/00* (2006.01)
*A23L 33/18* (2016.01)    *A23L 33/21* (2016.01)
*A61K 31/718* (2006.01)    *A61P 3/06* (2006.01)
*A61P 9/12* (2006.01)    *C07K 5/062* (2006.01)

(86) International application number:
**PCT/JP2019/005301**

(87) International publication number:
**WO 2019/160024 (22.08.2019 Gazette 2019/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.02.2018 JP 2018026410**
**16.02.2018 JP 2018026420**

(71) Applicant: **Morinaga Milk Industry Co., Ltd.**
**Minato-ku**
**Tokyo 108-8384 (JP)**

(72) Inventors:
• **TADA Asuka**
  **Kanagawa 2528583 (JP)**
• **TANAKA Miyuki**
  **Kanagawa 2528583 (JP)**
• **OCHI Daisuke**
  **Kanagawa 2528583 (JP)**
• **HORIGOME Miyako**
  **Kanagawa 2528583 (JP)**
• **YANAGISAWA Eiko**
  **Kanagawa 2528583 (JP)**

(74) Representative: **Vossius & Partner**
**Patentanwälte Rechtsanwälte mbB**
**Siebertstrasse 3**
**81675 München (DE)**

(54) **COMPOSITION FOR REDUCING BLOOD PRESSURE AND/OR FOR REDUCING NEUTRAL FATS**

(57)    Provided is a composition for lowering blood pressure and/or reducing neutral fat, which composition has few side effects and high safety. The composition for lowering blood pressure and/or reducing neutral fat includes a casein hydrolysate and an indigestible dextrin. Preferably, the casein hydrolysate includes a peptide composed of Met-Lys-Pro. Preferably, the casein hydrolysate includes a peptide composed of Met-Lys-Pro, and a content of the indigestible dextrin is at 5,000 to 100,000 parts by mass with respect to 1 part by mass of the peptide.

**Description**

Technical Field

The present technique relates to, for example, a composition for lowering blood pressure and/or reducing neutral fat.

Background Art

Since lifestyle-related diseases cause deterioration of health and longevity, the state and the medical institutions recommend improvement of the lifestyle to patients with lifestyle-related diseases and high-risk groups for these diseases. The lifestyle-related diseases can be divided into a number of groups including hypertension, dyslipidemia, and diabetes.

In hypertension, the vessel wall is placed under high pressure due to high blood pressure, causing damage to blood vessels. This leads to acceleration of the aging process, and produces adverse effects on the entire body (especially on, for example, the kidneys, brain, and retina of the eyes). In dyslipidemia, LDL cholesterol and neutral fat (triglyceride) in blood cause damage to the inside of blood vessels, gradually leading to arteriosclerosis. Although neither hypertension nor dyslipidemia exhibits characteristic symptoms, appearance of hypertension or dyslipidemia as an event may lead to death, or the event may cause deterioration of the quality of life (QOL).

Therefore, as a result, patients with hypertension, patients with dyslipidemia, and high-risk groups of these diseases (that is, borderline patients) require administration or ingestion of a substance that lowers blood pressure, a substance that reduces neutral fat or LDL cholesterol in blood, or the like. These individuals usually require long-term administration or ingestion of an effective component so as to maintain a state where the blood pressure is lowered, or where neutral fat or the like in blood is reduced. Therefore, the period during which high safety of the effective component can be achieved is preferably as long as possible. Further, from the viewpoint of prevention of development of the diseases, some people desire daily ingestion of the component when they have values only slightly higher than the standard values. Therefore, a highly safe effective component is demanded.

For example, JP-A-2001-252064 discloses that ingestion of indigestible dextrin has the same action as ingestion of dietary fiber, and that indigestible dextrin acts to slow down a sharp postprandial increase in the blood glucose, insulin, or neutral fat value.

Nevertheless, some people strongly desire an effective component having few side effects, high safety, and a better blood pressure-lowering action and/or neutral fat-reducing action.

Summary of the Invention

Problems to be Solved by the Invention

In view of this, a main object of the present technique is to provide a composition lowering blood pressure and/or reducing neutral fat, which composition has few side effects and high safety.

Means for Solving the Problems

In order to solve the above problems, the present inventors intensively studied to search for a substance having few side effects, high safety, and a better blood pressure-lowering action and/or neutral fat-reducing action. Indigestible dextrin is known to become in the form of a water-containing viscous gel, to inhibit absorption of components in the intestine. Therefore, the inventors once thought that the expected blood pressure-lowering action and/or neutral fat-reducing action can be hardly obtained even by long-term ingestion of indigestible dextrin in combination with other components.

However, the inventors discovered, in a model rat test, that an excellent blood pressure-lowering action and neutral fat-reducing action can be produced by combined ingestion of a mixture of a casein hydrolysate and indigestible dextrin, thereby completing the present invention.

More specifically, the present technique is as follows.

[1] A composition for lowering blood pressure and/or reducing neutral fat, comprising a casein hydrolysate and an indigestible dextrin.

[2] The composition according to [1], wherein the casein hydrolysate comprises a peptide of Met-Lys-Pro.

[3] The composition according to [1] or [2], comprising the indigestible dextrin and the casein hydrolysate at a mass ratio of 10:0.001 to 1:1.

[4] The composition according to any one of [1] to [3], wherein the casein hydrolysate comprises a peptide of Met-Lys-Pro, and an amount of the indigestible dextrin of 5,000 to 100,000 parts by mass with respect to 1 part by mass of the peptide.

[5] The composition according to any one of [1] to [4], wherein the composition is a pharmaceutical composition or a food or beverage composition.

[6] The composition according to [5], which is used for lowering blood pressure and/or for reducing neutral fat.

[7] The composition according to [5] or [6], which is used for the prevention or treatment of hypertension and/or dyslipidemia.

[8] The composition according to any one of [1] to [6], in the form of a unit package.

[9] The composition according to any one of [1] to [5], wherein the composition is a fermented food or beverage.

[10] The composition according to [9], wherein the fermented food or beverage comprises sucralose, and one or more monosaccharides and/or rare sugars selected from the group consisting of fructose, psicose, and allose.

[11] The composition according to [10], further comprising lactulose.

[12] A method of producing a fermented milk comprising a casein hydrolysate and an indigestible dextrin, and further comprising sucralose, and one or more monosaccharides and/or rare sugars selected from the group consisting of fructose, psicose, and allose.

[13] The method of producing a fermented milk according to [12], wherein the casein hydrolysate comprises a peptide of Met-Lys-Pro.

[14] The method of producing a fermented milk according to [12] or [13], wherein the fermented milk further comprises lactulose.

Effect of the Invention

According to the present technique, a composition for lowering blood pressure and/or reducing neutral fat, which composition has few side effects and high safety, can be provided. The effects described herein are not necessarily limited, and may be any of the effects described in the present technique.

Mode for Carrying Out the Invention

Preferred embodiments for carrying out the present technique are described below. The embodiments described below show examples of representative embodiments of the present disclosure. Interpretation of the scope of the present technique is not narrowed by these embodiments. In the present description, when a numerical range is expressed as "lower limit to upper limit", the upper limit may be either "or less" or "less than", and the lower limit may be either "or more" or "more than". In the present description, unless otherwise specified, percentage is expressed in terms of mass.

<1. Composition Having Blood Pressure-Lowering Action and/or Neutral Fat-Reducing Action According to Present Technique>

The present technique can exert a blood pressure-lowering action and/or neutral fat-reducing action by combination of a casein hydrolysate and an indigestible dextrin. The present technique is a composition for lowering blood pressure and/or reducing neutral fat, comprising a casein hydrolysate and an indigestible dextrin as effective components. This composition may be preferably used as a pharmaceutical composition or as a food or beverage composition (more preferably a fermented milk).

<1-1. Casein Hydrolysate>

The casein hydrolysate used in the present technique is obtained by hydrolysis of casein protein derived from milk. The casein hydrolysate contains various degraded components derived from the casein protein.

From the viewpoint of better production of the effect of the present technique, the casein hydrolysate preferably contains at least a peptide composed of Met-Lys-Pro (SEQ ID NO:1) (hereinafter also referred to as "tripeptide MKP").

The tripeptide MKP in the present technique is known to have effects such as an angiotensin converting enzyme-inhibiting action and a dipeptidyl-peptidase IV-inhibiting action (for example, Reference 1 (WO 2003/044044), Reference 2 (WO 2013/125622), and Reference 3 (JP-A-2016-069343)).

Preferably, the casein hydrolysate contains the tripeptide MKP, and satisfies one, or a combination of two or more, condition(s) selected from the following (a), (b), and (c):

(a) the average molecular weight of the casein hydrolysate is 1,200 daltons or less;

(b) the degradation rate of the casein hydrolysate is 20 to 30%; and

(c) the mass ratio of free amino acids in the total mass of all amino acids contained in the casein hydrolysate is 10% by mass or less.

The content of the tripeptide MKP in the casein hydrolysate is not limited. Regarding the lower limit of the content, from the viewpoint of better production of the effect of the present technique, the content is preferably 0.001% by mass or more, more preferably 0.005% by mass or more, still more preferably 0.01% by mass or more. Regarding the upper limit of the content, from the viewpoint of the production efficiency of the degradation product in the present technique, the content is preferably 1% by mass or less, more preferably 0.5% by mass or less, still more preferably 0.2% by mass or less, still more preferably 0.1% by mass or less. The content of the tripeptide MKP is more preferably 0.001 to 1% by mass, still more preferably 0.005 to 0.5% by mass, still more preferably 0.01 to 0.1% by mass.

The (a), (b), and (c) in the casein hydrolysate are preferably within the ranges achieved by the preparation in the "(2) Method of Producing Casein Hydrolysate" mentioned later.

<1-1-1. One Example of Method of Producing Casein Hydrolysate>

A method of producing the casein hydrolysate used in the present technique is described below in detail.

(1) Raw Material

The casein as a raw material contains milk-derived protein as a major component. The casein is not limited, and examples of the casein include commercially available caseins and caseinates. Specific examples of the casein include casein lactate, casein sulfate, casein hydrochloride, sodium caseinate, potassium caseinate, calcium caseinate, magnesium caseinate, and arbitrary mixtures thereof. Examples of the casein also include caseins and the like purified from cow's milk, skim milk, whole milk powder, or non-fat dry milk by a conventional method.

The casein hydrolysate as an effective component in the present technique is produced by hydrolysis of casein, which is a relatively inexpensive biomaterial, and which is a raw material derived from milk. Therefore, the casein hydrolysate can be produced stably and simply in a large amount.

(2) Method of Producing Casein Hydrolysate

The method of producing the casein hydrolysate is not limited, and examples of the method include a production method using an acid or alkali, and a production method using an enzyme such as protease. In particular, the use of an enzyme is preferred since it allows inclusion of a desired peptide and the like.

A method of producing a casein hydrolysate using protease is concretely described below. However, the present technique is not limited thereto.

First, a raw material (casein) is dispersed and dissolved in water.

The concentration of the solution is not limited. From the viewpoint of the efficiency and the workability, the concentration is usually preferably within the range of about 5 to 15% by mass in terms of protein.

Subsequently, the pH of the solution is adjusted to a pH close to the optimum pH of the protease employed, to prepare an aqueous raw material solution. More specifically, the pH of the solution is preferably adjusted using an alkaline solution to a pH of 7 to 10 since the optimum pHs of most proteases fall within this range.

The alkaline agent used for the pH adjustment is not limited, and examples of the agent include sodium hydroxide, potassium hydroxide, and potassium carbonate.

Subsequently, protease is added to the aqueous raw material solution prepared.

Examples of the protease include proteases derived from bacteria, animals, or plants. Any of these may be used.

Examples of the proteases derived from bacteria include, but are not limited to, endoproteases derived from the genus *Bacillus,* such as Alcalase (manufactured by Novo Nordisk), Neutrase (manufactured by Novozymes), Protin A (manufactured by Daiwa Fine Chemicals Co., Ltd.), Protin P (manufactured by Daiwa Fine Chemicals Co., Ltd.), Proleather (manufactured by Amano Enzyme Inc.), Protease N (manufactured by Amano Enzyme Inc.), Corolase 7089 (manufactured by Higuchi Shokai Co., Ltd.), Bioprase (manufactured by Nagase ChemteX Corporation), Orientase 90N (manufactured by HBI Enzymes Inc.), and Orientase 22BF (manufactured by HBI Enzymes Inc.).

Examples of the proteases derived from animals include, but are not limited to, proteases containing trypsin as a major component, such as PTN (manufactured by Novozymes) and trypsin V (manufactured by Biocon (Japan) Ltd.).

Examples of the proteases derived from plants include, but are not limited to, papain (manufactured by Amano Enzyme Inc.) and bromelain (manufactured by Amano Enzyme Inc.).

The proteases described above may be used individually, or as a combination of two or more thereof.

The protease is preferably dispersed and dissolved in cold water at 4 to 10°C before use. The concentration of the protease solution is not limited. From the viewpoint of the efficiency and the workability, the protease is usually preferably used in an amount with which the enzyme concentration becomes about 3 to 10%.

The amount of the protease used for the casein hydrolysis may vary depending on the substrate concentration, enzyme titer, reaction temperature, reaction time, and the like. In general, in a preferred mode, the ratio of the protease is 1000 to 20,000 units (activity units) per 1 g of casein in terms of the mass of protein.

The activity unit of the protease may be measured in accordance with the type of the protease employed.

When proteases are added, each kind of protease is preferably dissolved before the addition. The order of addition is not limited.

The temperature of the reaction system during the enzyme reaction is not limited, and can be selected within a practically applicable range including the optimum temperature range for production of the enzyme action. The temperature is usually selected within the range of 30 to 60°C.

Determination of the reaction duration may be difficult in some cases since the state of progress varies depending on reaction conditions such as the reaction temperature and the initial pH. For example, in cases where the reaction duration of the enzyme reaction is set at a constant, degradation products having different physicochemical properties among production batches may be produced, which is problematic. Therefore, it is preferred to monitor the enzyme reaction to determine the reaction duration such that desired values of physicochemical properties of the casein hydrolysate are achieved.

Examples of the monitoring method for the enzyme reaction include a method in which part of the reaction solution is collected, and subjected to measurement of the degradation rate of the protein or the like.

Subsequently, the enzyme reaction is stopped.

The enzyme reaction is stopped by deactivation of the enzyme in the hydrolysate. The deactivation treatment may be carried out by a conventional method such as heat deactivation treatment.

The conditions for the heat deactivation treatment (such as the heating temperature and the heating time) may be appropriately set such that the deactivation can be sufficiently achieved, taking into account the thermal stability of the enzyme employed. The heat deactivation may be carried out, for example, within the temperature range of 80 to 130°C for a retention time of 30 minutes to 2 seconds.

After the stopping of the enzyme reaction, the resulting hydrolyzed deactivated liquid is preferably purified by a method, or a combination of two or more methods, such as (1) filtration; (2) membrane separation treatment with a microfiltration membrane, ultrafiltration membrane, or the like; and (3) resin adsorption separation.

By carrying out the purification, removal of the insoluble matter contained in the deactivated liquid, reduction of fat, lactose, and other unnecessary components, and the like are possible. As a result, a casein hydrolysate which is transparent in the solution state, and which does not cause cloudiness, precipitation, aggregation, browning, or the like during a long-term storage in the solution state, that is, which has excellent shelf stability, can be obtained.

Further, by carrying out the purification, the casein hydrolysate used in the present technique can have improved taste, external appearance, and the like.

The filtration of (1) may be carried out by a known method. For example, the filtration may be carried out using diatomaceous earth with a known apparatus.

By carrying out the filtration, the insoluble matter present in the hydrolyzed deactivated liquid, produced during the hydrolysis reaction and/or the enzyme heat deactivation, can be removed.

The membrane separation treatment of (2) may be carried out using a known apparatus. Examples of the known apparatus include, but are not limited to, microfiltration modules; and ultrafiltration modules such as SEP1053 (manufactured by Asahi Kasei Corporation; molecular weight cutoff, 3,000), SIP1053 (manufactured by Asahi Kasei Corporation; molecular weight cutoff, 6,000), and SLP1053 (manufactured by Asahi Kasei Corporation; molecular weight cutoff, 10,000).

In this case, a solution containing the casein hydrolysate is obtained as a membrane permeate fraction after the membrane separation treatment.

By carrying out the membrane separation treatment, similar to the filtration of (1), the insoluble matter present in the hydrolyzed deactivated liquid, produced during the hydrolysis reaction and/or the enzyme heat deactivation, can be removed.

The resin adsorption separation of (3) may be carried out by a known method, for example, by packing a column with a resin, and then passing the hydrolyzed deactivated liquid through the column. Examples of the resin include, but are not limited to, trade names: Diaion and Sepabeads (manufactured by manufactured by Mitsubishi Chemical Corporation), Amberlite XAD (manufactured by Organo Corporation), and KS-35 (manufactured by Ajinomoto Fine-Techno Co., Inc.).

The resin adsorption separation may be carried out by a continuous method, in which a column is packed with the resin, and then the hydrolyzed deactivated liquid is continuously allowed to flow therethrough, or by a batch method, in which the resin is fed into the hydrolyzed deactivated liquid so as to bring the resin into contact with the hydrolyzed deactivated liquid for a certain length of time, and then the hydrolyzed deactivated liquid is separated from the resin.

The hydrolyzed deactivated liquid may contain residual factors (such as peptides containing a large amount of hydrophobic amino acids) that cause clouding, precipitation, aggregation, browning, or the like during the storage period.

These factors can be removed by carrying out the resin adsorption separation.

Further, the purification may be followed by sterilization of the resulting solution containing the casein hydrolysate.

The sterilization method may be, for example, a conventional heat treatment method.

Regarding the heating temperature and the retention time during the heat treatment, appropriate conditions that allow the sterilization may be appropriately set. For example, the sterilization may be achieved by heat treatment at 70 to 140°C for 2 seconds to 30 minutes.

The method of the heat sterilization may be either a batch method or a continuous method. For the continuous method, methods such as a plate heat exchange method, an infusion method, and an injection method may be used.

The solution containing the casein hydrolysate obtained may be used as it is, or may be, when necessary, used as a concentrated liquid prepared by concentrating the solution by a known method. Alternatively, the concentrated liquid may dried by a known method to prepare a powder, and the powder may be used.

Further, after the removal of the insoluble matter components from the casein hydrolysate, secondary hydrolysis may be carried out using an endoprotease or exoprotease for the purpose of improvement of the taste, improvement of the physical properties, or the like.

Further, after the removal of the insoluble matter components from the casein hydrolysate, secondary hydrolysis may be carried out using an endoprotease or exoprotease for the purpose of improvement of the taste, improvement of physical properties, or the like.

The casein hydrolysate used in the present technique is preferably prepared such that it has an average molecular weight of preferably 1,200 daltons or less, more preferably 1,000 daltons or less, still more preferably 800 daltons or less, still more preferably 450 daltons or less, from the viewpoint of better production of the effect of the present technique. The preparation is preferably carried out to achieve an average molecular weight of more preferably 250 to 450 daltons, still more preferably 360 to 390 daltons.

The casein hydrolysate used in the present technique is preferably prepared such that it has a degradation rate of preferably 10% or more regarding the lower limit, and preferably 40% or less regarding the upper limit; more preferably prepared such that it has a degradation rate of 20 to 30%, from the viewpoint of better production of the effect of the present technique.

In the present technique, reaction conditions such as the reaction temperature and the reaction duration are preferably determined so as to set the degree of hydrolysis such that the casein hydrolysate contained in the filtrate after the removal of the insoluble matter produced due to the hydrolysis has an average molecular weight within a desired range, and/or such that the casein hydrolysate has a degradation rate within a desired range.

The casein hydrolysate used in the present technique is more preferably prepared such that the ratio of the total mass of free amino acids in the total mass of all amino acids contained therein is preferably 15% by mass or less more preferably 10% by mass or less, from the viewpoint of better production of the effect of the present technique.

The casein hydrolysate used in the present technique is preferably prepared such that the ratio of the tripeptide MKP contained therein is preferably 0.001 to 1% by mass, more preferably 0.005 to 0.5% by mass, still more preferably 0.01 to 0.1% by mass, from the viewpoint of the effect and the production efficiency.

In the present technique, the ratio of the total mass of free amino acids or the ratio of the tripeptide MKP may be adjusted such that each desired ratio can be achieved, by, for example, selecting the type of the enzyme for the hydrolysis of casein, the amount of the enzyme added, the reaction time, and/or purification conditions (membrane separation, resin adsorption separation, or the like) after the hydrolysis.

The <Degradation Rate of Amino Acids>, <Method of Calculating Average Molecular Weight>, <Method of Calculating Free Amino Acid Ratio>, and <Measurement of Tripeptide MKP Content> in the casein hydrolysate of the present technique are described below.

<Method of Calculating Molecular Weight>

The average molecular weight (Da: dalton) of the casein hydrolysate of the present technique is calculated according to the following concept of number average molecular weight.

For example, as described in The Society of Polymer Science, Japan (ed.) Basic High Polymer Science. pp. 116 to 119 (Tokyo Kagaku Dojin Co. Ltd., 1978), the number average molecular weight (Number Average of Molecular Weight) represents the average value of the molecular weight of a macromolecular compound based on a distinct index as follows.

More specifically, since a macromolecular compound such as a protein hydrolysate is a heterogeneous substance, and has a molecular weight distribution, the molecular weight of a protein hydrolysate needs to be expressed as an average molecular weight for the purpose of physicochemical approaches. The number average molecular weight (which may be hereinafter simply referred to as Mn) is the average in terms of the number of molecules, and defined by the following Equation (1), wherein the molecular weight of a peptide chain i is represented as Mi, and its number of molecules is represented as Ni.
Equation 1:

$$\mathrm{Mn} = \sum_{i=1}^{\infty} \mathrm{Mi\ Ni} \Big/ \sum_{i=1}^{\infty} \mathrm{Ni} \quad \cdots (1)$$

<Method of Calculating Degradation Rate>

The degradation rate of the casein hydrolysate can be calculated according to the following Equation (2). The amount of formol nitrogen can be determined by the formol method, and the total amount of nitrogen can be determined by the Kjeldahl method.
Equation 2:

$$\text{Degradation rate (\%)} = (\text{amount of formol nitrogen} \,/\, \text{total amount of nitrogen})$$

$$\times 100 \ldots (2)$$

<Method of Calculating Free Amino Acid Ratio>

In the present technique, the ratio of the total mass of free amino acids can be calculated according to, for example, the following procedure.

(a) Measurement of Amino Acid Composition

For amino acids other than tryptophan, cysteine, and methionine, the sample is hydrolyzed with 6 N hydrochloric acid at 110°C for 24 hours. For tryptophan, the sample is subjected to alkaline degradation with barium hydroxide at 110°C for 22 hours. For cysteine and methionine, the sample is treated with performic acid, and then hydrolyzed with 6 N hydrochloric acid at 110°C for 18 hours. Each resulting product is analyzed using an amino acid analyzer (for example, Type 835, manufactured by Hitachi, Ltd.) to measure the mass of the amino acid.
In this method, the amounts of glutamine and glutamic acid in the sample are quantified as the glutamic acid analysis value, which is their total amount.

(b) Calculation of Ratio of Total Mass of Free Amino Acids

The composition of each amino acid in the sample is measured by the method of (a) measurement of the amino acid composition, and the total of the measured values is calculated as the mass of all amino acids in the sample. Subsequently, the sample is subjected to deproteinization using sulfosalicylic acid, and the mass of each residual free amino acid is measured by the method of (a). The total of the measured values is calculated as the mass of all free amino acids in the sample. From these values, the ratio of the total mass of free amino acids in the sample is calculated according to the following Equation (3).
Equation 3:

$$\text{Ratio of total mass of free amino acids (\%)} = (\text{mass of all free amino acids} \,/$$

$$\text{mass of all amino acids}) \times 100 \ldots (3)$$

<Measurement of Tripeptide MKP Content>

(a) A sample powder is diluted/dissolved in 0.2% aqueous formic acid solution at 1.0 mg/mL, and the resulting solution is sonicated for 10 minutes, followed by filtration through a PVDF filter (manufactured by Millipore) having a pore size of 0.22 $\mu$m, to prepare a powder solution. LC/MS analysis is carried out under the following measurement conditions. On the other hand, several solutions of a chemically synthesized reference peptide of the tripeptide MKP (manufactured by Peptide Institute, Inc.) having different concentrations are prepared, and LC/MS analysis is carried out under the following measurement conditions to prepare a calibration curve.

Among the peaks in the analysis of the powder solution, a peak having the same molecular weight and retention time is identified as having the same sequence as the reference peptide. By comparison between the peak area of the reference peptide and the peak area of the sample powder, the content of the tripeptide MKP in the powder solution is determined.

(b) MKP Content (mg/l g of Casein Hydrolysate)

$$\text{MKP content (mg/1 g of casein hydrolysate)} = [\text{measured value of tripeptide}$$
$$\text{MKP (mg) in casein hydrolysate obtained}] / [\text{mass (g) of casein hydrolysate obtained}]$$

The [measured value of tripeptide MKP (mg) in casein hydrolysate obtained] is a measured value of the tripeptide MKP in the sample according to the following "LC/MS".

(c) LC/MS Apparatus Used

Mass spectrometer: TSQ Quantum Discovery MAX (manufactured by Thermo Fisher Scientific Inc.).

High-performance liquid chromatography: Prominence (manufactured by Shimadzu Corporation); column: XBridge BEH300 C18 2.1 mm (diameter) $\times$ 250 mm, 3.5 $\mu$m (manufactured by Waters).

(d) LC/MS Measurement Conditions

Mobile phase A: 0.2% by weight aqueous formic acid solution
Mobile phase B: 0.2% by weight formic acid - acetonitrile solution

Time Program: 2% B (0.0 min) - 25% B (5.0 min) - 65% B (5.1 min) - 65% B (10 min) - 85% B (10.1 min) - 85% B (13.0%) - 2% B (13.1 min) - STOP (30.0 min).

Sample Injection Volume: 10 $\mu$L; Column temperature: 40°C; Liquid flow rate: 200 $\mu$L/min

Analysis mode: SRM measurement

Product Mass: m/z = 260.10 (Parent m/z = 375.21)

<1-2. Indigestible Dextrin>

The indigestible dextrin used in the present technique is a water-soluble dietary fiber obtained from starch, and may be an indigestible dextrin which can be obtained, for example, by enzymatic digestion of pyrodextrin, or by enzymatic digestion followed by hydrogenation.

The indigestible dextrin in the present technique means a water-soluble dietary fiber having an indigestible property, prepared by subjecting starch derived from a plant such as corn, wheat, rice, beans, tubers, or tapioca to acid addition (for example, addition of hydrochloric acid) and/or heating, to obtain a pyrodextrin, and then, when necessary, subjecting the resulting pyrodextrin to enzyme treatment with $\alpha$-amylase and/or glucoamylase, and then, when necessary, subjecting the treated product to desalting and decoloring. Examples of the indigestible dextrin include: dextrins containing an indigestible component measured according to high-performance liquid chromatography (enzyme-HPLC method) which is a method for analyzing dietary fiber described in EISHIN No.13, April 26, 1999 (Analytical Methods for Nutrients on the Standards for Nutrition Labeling); preferably dextrins containing 85 to 95% by weight indigestible component. Examples of the indigestible dextrin used in the invention also include reduced products of indigestible dextrin produced by hydrogenation, for convenience.

Indigestible dextrins and reduced products thereof (reduced indigestible dextrins) are commercially available in the forms of powders, fine granules, granules, and the like. Any of these forms may be used in the present technique.

In the present description, the term "indigestible" in indigestible dextrin means that the dextrin is hardly digestible by human digestive enzymes.

The indigestible dextrin may be quantified by high-performance liquid chromatography (enzyme-HPLC method (AOAC 2001.03)) (for example, Reference 4 (JP-A-2001-252064), Reference 5 (Indigestible Fractions of Starch Hydrolysates and Their Determination Method. Kazuhiro Okuma and Isao Matsuda. J. Appl. Glycosci., Vol. 49, No. 4, p. 479-485 (2002)), Reference 6 (Pyrolysis of Starch and Its Digestibility by Enzymes -Characterization of Indigestible Dextrin-, Kazuhiro Ohkuma, Isao Matsuda, Yasuo Katta, and Yoshio Hanno. Denpun kagaku Vol. 37, No. 2, p. 107-114 (1990)), and Reference 7 (Production of Indigestible Dextrin from Pyrodextrin. Kazuhiro Ohkuma, Isao Matsuda. J. Appl. Glycosci., Vol. 50 No. 3 p. 389-394 (2003)).

The indigestible dextrin is a low-calorie, low-fat food material. It is said to have physiologically active effects such as an intestinal regulation action, blood glucose elevation-suppressing action, serum cholesterol-lowering action, intestinal environment-improving action, and neutral fat-lowering action (for example, Reference 8 (JP-A-2001-252064) and Reference 9 (JP-A-4-91765)).

For the method of producing the indigestible dextrin in the present technique, one may refer to, for example, Reference 8 and Reference 9. Examples of the starch as a raw material of the indigestible dextrin include, but are not limited to, starches of corn, potato, sweet potato, tapioca, wheat, barley, and rice. Among these, corn starch is preferred. Examples of the mineral acid for the acid addition include hydrochloric acid, nitric acid, and sulfuric acid. Among these, hydrochloric acid is preferred.

<1-3. Use of Casein Hydrolysate and Indigestible Dextrin in Present Technique>

As described later in the Examples, combined use of the casein hydrolysate and the indigestible dextrin in the present technique has a blood pressure-lowering action and/or neutral fat-reducing action. Therefore, they can be used for prevention, amelioration, and/or treatment of hypertension and/or dyslipidemia (hyperlipidemia). Further, the casein hydrolysate and the indigestible dextrin in the present technique are effective for individuals with these diseases or symptoms, or for high-risk groups thereof. In particular, the present technique may be used for prevention, amelioration, or treatment of arteriosclerosis, wherein progression of symptoms tends to occur as a complication of hypertension and dyslipidemia.

It has been generally suggested that diuretics used as hypotensive drugs may cause hyperglycemia, and there is a report that glucosyl hesperidin, which is reported to have a blood neutral fat-reducing action, has not been shown to have an effect on the postprandial blood glucose level. However, Reference 10 (JP-A-2017-31105) reports that combined use of a casein hydrolysate and an indigestible dextrin has an effect that suppresses an increase in the postprandial blood glucose level. More specifically, although it has been conventionally reported that blood pressure-lowering compositions or neutral fat-reducing compositions may have the risk of hyperglycemia or exhibit no suppression of the postprandial blood glucose level, the casein hydrolysate and the indigestible dextrin in the present technique can provide an excellent blood pressure-lowering and/or neutral fat-reducing composition which has only a low risk of causing hyperglycemia, which exhibits all of a blood pressure-lowering action, a neutral fat-reducing action, and suppression of the postprandial blood glucose elevation, and which has a synergistic effect that could not be conventionally achieved. The present technique can provide a blood pressure-lowering and/or neutral fat-reducing composition or formulation having a reduced risk of side effects for, for example, patients with hypertension or abnormal lipid metabolism.

Thus, the casein hydrolysate and the indigestible dextrin in the present technique may be included, as an effective component, in a composition or formulation for lowering blood pressure and/or reducing neutral fat, or in a composition or formulation for hypertension and/or dyslipidemia (hyperlipidemia). Further, they may be used for a variety of applications such as pharmaceuticals, foods and beverages, and feeds. Alternatively, they may be materials or formulations to be blended with these foods and beverages, and the like.

The casein hydrolysate and the indigestible dextrin in the present technique may be used as they are for animals including humans. Alternatively, they may be used as a mixture prepared with a sitologically or pharmaceutically acceptable ordinary carrier or diluent. By this, the effects of the present technique can be produced.

Further, the casein hydrolysate and the indigestible dextrin in the present technique may be used for production of these compositions or formulations.

The present embodiment may be either used for a therapeutic purpose or used for a non-therapeutic purpose.

The "non-therapeutic purpose" is a concept which does not include medical actions, that is, actions of treatment carried out for human bodies by therapies. The concept includes, for example, health promotion and cosmetic actions.

The "amelioration" means improvement of a disease, symptom, or condition; prevention or slowing of exacerbation of a disease, symptom, or condition; or reversal, prevention, or slowing of progression of a disease or symptom.

The "prevention" means prevention or slowing of development of a disease or symptom in a subject of application, or lowering of the risk of a disease or symptom in a subject of application.

The casein hydrolysate and the indigestible dextrin according to the present technique; and a composition for lowering blood pressure and/or reducing neutral fat, a composition that lowers the blood pressure and/or reduces neutral fat, and a composition for prevention, amelioration, and/or treatment of hypertension and/or dyslipidemia (hyperlipidemia), containing these (hereinafter referred to as "composition or the like"); may be used for any of pharmaceuticals, foods and beverages, and feeds (preferably pet foods), for utilization of their excellent blood pressure-lowering action and/or neutral fat-reducing action.

Since the casein hydrolysate and the indigestible dextrin used in the present technique are readily soluble in water, it has excellent processing stability. Further, since they produce the effect of the present technique through oral ingestion, they are preferably used for oral ingestion.

The form of the composition of the present technique is not limited, and the composition is preferably in the form of a powder or granule, a tablet formed by compression, or a capsulated formulation containing the composition filled therein.

The composition of the present technique may be in the form of a unit package. The composition in the form of a unit package is not limited as long as the two components in the present technique are contained in the unit package. For example, the composition may be contained in the unit package in the form of separate formulations or compositions such as a formulation containing the casein hydrolysate and a formulation containing the indigestible dextrin, or may be contained in the unit package in the form of a mixture in the powder state containing the two components in the present technique. From the viewpoint of convenience, the unit package is preferably in the form of a unit package for each meal.

The content or the amount of the casein hydrolysate used for the composition or the like according to the present technique may be arbitrarily set in accordance with the age, symptoms, and the like as long as the effect of the present technique is not deteriorated.

In the present technique, the amount of the casein hydrolysate may be set to 0.001 to 1% by mass in the final composition.

In the present technique, the amount of the tripeptide MKP may be set to 0.00001 to 0.005% by mass in the final composition.

In the present description, the final composition means the composition to be administered to the subject or to be ingested by the subject.

In the present technique, the amount of the casein hydrolysate provided to the subject is preferably 0.0001 to 1 g/kg body weight/day, more preferably 0.0005 to 0.5 g/kg body weight/day, still more preferably 0.0001 to 0.1 g/kg body weight/day.

The content or the amount of the indigestible dextrin used in the present technique may be arbitrarily set in accordance with the age, symptoms, and the like as long as the effect of the present technique is not deteriorated.

In the present technique, for example, in cases where the amount of the indigestible dextrin for each meal is about 5 g, the amount of the indigestible dextrin may be set to 1 to 10% by mass in the final composition.

In the present technique, the amount of the indigestible dextrin used for the subject is preferably 0.001 to 10 g/kg body weight/day, more preferably 0.005 to 5 g/kg body weight/day, still more preferably 0.01 to 1 g/kg body weight/day.

The content or the amount of each of the casein hydrolysate and the indigestible dextrin used in the present technique is not limited. From the viewpoint of better production of the effect of the present technique, the ratio between their contents or amounts is preferably indigestible dextrin : casein hydrolysate = 10:0.001 to 1:1, more preferably indigestible dextrin : casein hydrolysate = 1:0.001 to 10:1.

The content or the amount of each of the tripeptide MKP and the indigestible dextrin used in the present technique is not limited. From the viewpoint of better production of the effect of the present technique, the content or the amount of the indigestible dextrin used with respect to 1 part by mass of the tripeptide MKP is, regarding the lower limit, preferably 5,000 parts by mass or more, more preferably 10,000 parts by mass or more, and, regarding the upper limit, preferably 100,000 parts by mass or less, more preferably 80,000 parts by mass or less. Within this range, the content or the amount of the indigestible dextrin is preferably 15,000 to 50,000 parts by mass.

In the present technique, the casein hydrolysate and the indigestible dextrin may be used either in the same time period or separately, as long as the effect of the present technique is not deteriorated.

The interval of use (more specifically, the interval of administration or ingestion) in the present technique is not limited, and the technique may be carried out once a day, or dividedly in several times per day. The present technique is preferably continuously used (more specifically, administered or ingested) every day.

Since, empirically, the casein hydrolysate and the indigestible dextrin used in the present technique are highly safe, they can be continuously ingested for a long period. The present technique is excellent from the viewpoint of the fact that, by using it continuously for a long period, the prevention effect of the present technique can be continuously produced. It is thus preferably used every day continuously for, for example, 30 days or longer.

The subject of the present technique is not limited, and may be a human or an animal other than humans. Since the two components in the present technique are highly safe, and can be ingested for a long period, the age and sex of the subject are not limited.

Since the present technique provides the blood pressure-lowering action and/or neutral fat-reducing action, the subject is preferably an individual who expects lowering of the blood pressure and/or reduction of neutral fat, or an individual who has the risk of, or who is at high risk of, hypertension and/or dyslipidemia,

<1-3-1. Pharmaceuticals and Quasi-Drugs>

For utilization of the excellent effect, the present technique may be used by being blended, as an effective component, in a pharmaceutical, quasi-drug, or the like (hereinafter referred to as "pharmaceutical or the like") for humans or animals, to be used for lowering of the blood pressure and/or reduction of neutral fat, and/or for prevention, amelioration, and/or treatment of hypertension and/or dyslipidemia (hyperlipidemia).

In the cases of blending in a pharmaceutical or the like, the pharmaceutical may be appropriately formulated into a desired dosage form in accordance with the administration method such as oral administration or parenteral administration. The dosage form is not limited, and, in the cases of oral administration, the pharmaceutical may be formulated into, for example, a solid formulation such as a powder, granule, tablet, troche, or capsule; or a liquid formulation such as a solution, syrup, suspension, or emulsion. In the cases of parenteral administration, the pharmaceutical may be formulated into, for example, a suppository, spray, inhalant, ointment, patch, or injection solution. In the present technique, the pharmaceutical is preferably formulated into a formulation for oral administration.

The formulation may be appropriately carried out by a known method in accordance with the dosage form.

The formulation may be carried out by, for example, blending with a formulation carrier as appropriate. In addition to the peptide in the present technique, components normally used for formulation, such as excipients, pH adjusting agents, coloring agents, and corrigents may be used. Further, components effective for prevention, amelioration, and/or treatment of diseases and/or symptoms which are known, or which may be found in the future, may be used in combination as appropriate depending on the purpose.

As the formulation carrier, various organic or inorganic carriers may be used depending on the dosage form. Examples of carriers for the cases of a solid formulation include excipients, binders, disintegrators, lubricants, stabilizers, and correctives.

Examples of the excipients include sugar derivatives such as lactose, sucrose, glucose, mannitol, and sorbitol; starch derivatives such as corn starch, potato starch, $\alpha$-starch, dextrin, and carboxymethyl starch; cellulose derivatives such as crystalline cellulose, hydroxypropyl cellulose, hydroxypropyl methyl cellulose, carboxymethyl cellulose, and carboxymethyl cellulose calcium; gum arabic; dextran; pullulan; silicate derivatives such as light anhydrous silicic acid, synthetic aluminum silicate, and magnesium aluminometasilicate; phosphate derivatives such as calcium phosphate; carbonate derivatives such as calcium carbonate; and sulfate derivatives such as calcium sulfate.

Examples of the binders include, in addition to the excipients described above, gelatin; polyvinylpyrrolidone; and Macrogol.

Examples of the disintegrators include, in addition to the excipients described above, chemically modified starch or cellulose derivatives such as croscarmellose sodium, sodium carboxymethyl starch, and cross-linked polyvinylpyrrolidone.

Examples of the lubricants include talc; stearic acid; metal stearates such as calcium stearate and magnesium stearate; colloidal silica; waxes such as Veegum and spermaceti; boric acid; glycol; carboxylic acids such as fumaric acid and adipic acid; sodium carboxylates such as sodium benzoate; sulfates such as sodium sulfate; leucine; lauryl sulfates such as sodium lauryl sulfate and magnesium lauryl sulfate; silicic acids such as anhydrous silicic acid and silicic acid hydrate; and starch derivatives.

Examples of the stabilizers include paraoxybenzoic acid esters such as methylparaben and propylparaben; alcohols such as chlorobutanol, benzyl alcohol, and phenylethyl alcohol; benzalkonium chloride; acetic anhydride; and sorbic acid.

Examples of the correctives include sweeteners, acidulants, and flavoring agents.

Examples of the carrier used in the cases of a liquid for oral administration include solvents such as water; and correctives.

The amount of the casein hydrolysate used for the pharmaceutical or the like according to the present technique may be arbitrarily set in accordance with the age, symptoms, and the like as long as the effect of the present technique is not deteriorated. In particular, in the present technique, the amount of the casein hydrolysate may be set to 0.001 to 1% by mass for the final composition such as a pharmaceutical.

In the present technique, the amount of the casein hydrolysate used for the subject is preferably 0.0001 to 1 g/kg body weight/day, more preferably 0.0005 to 0.5 g/kg body weight/day, still more preferably 0.001 to 0.1 g/kg body weight/day.

The amount of the indigestible dextrin used for the pharmaceutical or the like according to the present technique may also be arbitrarily set in accordance with the age, symptoms, and the like as long as the effect of the present technique is not deteriorated. In the present technique, for example, in cases where the amount of the indigestible dextrin for each meal is about 5 g, the amount of the indigestible dextrin may be set to 1 to 10% by mass for the final composition such as a pharmaceutical.

In the present technique, the amount of the indigestible dextrin used for the subject is preferably 0.001 to 10 g/kg body weight/day, more preferably 0.005 to 5 g/kg body weight/day, still more preferably 0.01 to 1 g/kg body weight/day.

Thus, in the composition provided in the form of a pharmaceutical or the like, the content or the amount of each of the indigestible dextrin and the casein hydrolysate used is not limited. From the viewpoint of better production of the effect of the present technique, the mass ratio between their contents or amounts used is preferably indigestible dextrin : casein hydrolysate = 10:0.001 to 1:1, more preferably indigestible dextrin : casein hydrolysate = 1:0.001 to 10:1.

The content or the amount of each of the tripeptide MKP and the indigestible dextrin used in the present technique is not limited. From the viewpoint of better production of the effect of the present technique, the content or the amount of the indigestible dextrin used with respect to 1 part by mass of the tripeptide MKP is, regarding the lower limit, preferably 5,000 parts by mass or more, more preferably 10,000 parts by mass or more, and, regarding the upper limit, preferably 100,000 parts by mass or less, more preferably 80,000 parts by mass or less. Within this range, the content or the amount of the indigestible dextrin is preferably 15,000 to 50,000 parts by mass.

<1-3-2. Food or Beverage>

For utilization of the excellent effect, the present technique may be used by being blended, as an effective component, in foods and beverages for humans or animals; health foods; functional foods; patient foods; enteral nutrition foods; foods for special dietary uses; foods with health claims; foods for specified health uses; foods with functional claims whose labels indicate their usefulness in lowering of the blood pressure and/or reduction of neutral fat, and/or in prevention, amelioration, and/or treatment of hypertension and/or dyslipidemia (hyperlipidemia); foods with nutrient function claims; and the like; to be used for lowering of the blood pressure and/or reduction of neutral fat, and/or for prevention, amelioration, and/or treatment of hypertension and/or dyslipidemia (hyperlipidemia) (hereinafter also referred to as "food, beverage, or the like").

The present technique may be prepared by addition to a known food or beverage, or a novel food or beverage may be produced by mixing with a raw material(s) of a food or beverage.

The food, beverage, or the like may be in any form such as a liquid, paste, solid, or powder. Examples of the food, beverage, or the like include tablet confectioneries, liquid diets, and feeds (including those for pets), and also include flour products, instant foods, processed agricultural products, processed fishery products, processed livestock products, milk and milk products, fats and oils, basic seasonings, composite seasonings and foods, frozen foods, confectioneries, beverages, and other commercially available products.

Examples of the flour products include bread, macaroni, spaghetti, noodles, cake mixes, deep frying flour, and bread crumbs.

Examples of the instant foods include instant noodles, cup noodles, retort/cooked foods, canned cooked foods, microwave oven foods, instant soups and stews, instant miso soups and Japanese clear soups, canned soups, freeze-dried foods, and other instant foods.

Examples of the processed agricultural products include canned agricultural products, canned fruits, jam and marmalade, pickles, boiled beans, dried agricultural products, and cereals (processed cereal products).

Examples of the processed fishery products include canned fishery products, fish ham/sausage, fish-paste products, marine delicacies, and tsukudani.

Examples of the processed livestock products include canned products and pastes of livestock; and livestock meat ham and sausage.

Examples of the milk and milk products include processed milk, milk beverages, yogurt, lactic acid bacteria beverages, cheese, ice cream, modified milk powder, cream, and other milk products.

Examples of the fats and oils include butter, margarine, and vegetable oils.

Examples of the basic seasonings include soy sauce, miso, sauces, processed tomato seasonings, sweet sake ("mirin"), and vinegar. Examples of the composite seasonings and foods include cooking mixes, curry bases, sauces ("tare"), dressings, noodle soup bases ("mentsuyu"), spices, and other composite seasonings.

Examples of the frozen foods include frozen food materials, half-cooked frozen foods, and cooked frozen foods.

Examples of the confectioneries include caramels, candies, chewing gums, chocolates, cookies, biscuits, cakes, pies, snacks, crackers, Japanese sweets, rice biscuits, beans confectioneries, desserts, and other confectioneries.

Examples of the beverages include carbonated beverages, natural fruit juices, fruit juice beverages, soft drinks containing fruit juices, fruit pulp beverages, berry-containing fruit beverages, vegetable-containing beverages, soybean milk, soybean milk beverages, coffee beverages, tea beverages, powdered beverages, concentrated beverages, sports beverages, nutritional beverages, alcoholic beverages, and other beverages of taste.

Examples of the other commercially available products include baby foods, rice seasonings ("furikake"), and ochazuke-nori (dried lavar for boiled rice soaked in green tea).

1-3-2-1. Method of Producing Food or Beverage of Present Technique

The food or beverage of the present technique may be produced by adding a casein hydrolysate and an indigestible dextrin to a raw material(s) of a food or beverage composition. The production may be carried out in the same manner as production of ordinary food or beverage compositions except that the casein hydrolysate and the indigestible dextrin are added. The casein hydrolysate and the indigestible dextrin in the present technique may be added at any stage in the production process of the food or beverage composition.

The food, beverage, or the like of the present technique may contain a beneficial bacterium. Among the foods and beverages containing beneficial bacteria, fermented foods and beverages are preferred from the viewpoint of adding a probiotic effect due to fermentation by a beneficial bacterium.

In the method of producing the fermented food or beverage, the casein hydrolysate and the indigestible dextrin may be added at any stage in the production process. For example, the fermented food or beverage may be produced by including them as raw materials and performing a fermentation step. Examples of such a food or beverage include lactic acid bacteria beverages and fermented milks.

1-3-2-2. Fermented Food or Beverage of Present Technique, and Production Method Thereof

The method of producing a fermented food or beverage of the present technique is described below. However, the method is not limited thereto.

In the method of producing a fermented food or beverage of the present technique, a casein hydrolysate and an indigestible dextrin may be used as raw materials of the fermented food or beverage. The casein hydrolysate and/or the indigestible dextrin in the present technique may be used before the fermentation step and/or after the fermentation step for the fermented food or beverage.

Unless otherwise specified, the raw materials used in the production method of the present technique are raw materials that may be used in any step in the method of producing a fermented milk, and may be used as pre-fermentation raw materials and/or post-fermentation raw materials. As the raw materials, those described later in

<2. Method of Producing Fermented Milk Containing Milk Protein Hydrolysate and/or Indigestible Dextrin> may be used.

From the viewpoint of better production of effects of the present technique such as lowering of the blood pressure and/or reduction of neutral fat, one or more of low-calorie sweeteners, rare monosaccharides, and milk oligosaccharides are preferably used as the raw materials. Further, (1) sucralose; and (2) one or more monosaccharides and/or rare sugars such as fructose, psicose, and allose; are preferably used in combination. The raw materials more preferably include (3) lactulose in addition to (1) and (2).

From the viewpoint of improvement of the taste, a low-calorie sweetener and/or a rare sugar is/are preferably used, a combination of a low-calorie sweetener and a rare sugar is more preferably used, as a raw material(s). Further, from the viewpoint of improvement of the taste, the combination of (1) sucralose; and (2) one or more monosaccharides and/or rare sugars such as fructose, psicose, and allose; is more preferably used. Still more preferably, (3) lactulose can be used in addition to (1) and (2).

The "rare sugar" generally means a monosaccharide or sugar alcohol which is present in a small amount in nature.

A milk oligosaccharide (preferably lactulose) is preferably used as a raw material from the viewpoint of giving a milk taste, from the viewpoint of delivery to the large intestine without degradation or absorption, to thereby provide an assimilable component for bifidobacteria, and from the viewpoint of promoting absorption of calcium and magnesium. The content of the low-calorie sweetener is preferably 0.001 to 0.1% by mass in the final product. The content of the rare sugar is preferably 0.005 to 0.25% by mass in the final product. The content of the lactulose is preferably 0.5 to 6% by mass in the final product.

Examples of the low-calorie sweetener include aspartame, acesulfame K, sucralose (another name: 4,1',6'-trichlorogalactosucrose), stevia sweeteners, saccharin, sodium saccharinate, and licorice extracts. Their commercially available products may also be used. One or more of these low-calorie sweeteners may be selected. Among these, sucralose is preferred from the viewpoint of improvement of the taste, and the effect of the present technique.

Among the rare sugars described above, examples of rare monosaccharides include D-psicose and D-allose. Among the rare sugars, examples of rare sugar alcohols include xylitol and erythritol. Their commercially available products may also be used. Examples of the commercially available products containing D-psicose, D-allose, or the like include a rare sugar-containing syrup (Rare Sugar Sweet (manufactured by Matsutani Chemical Industry Co., Ltd.)). This rare sugar-containing syrup contains fructose, D-psicose, and D-allose. One or more of these rare sugars may be selected. Among these, rare monosaccharides are preferred. Further, among the rare monosaccharides, D-psicose and/or D-allose is/are preferred. Combined use of D-psicose and D-allose is more preferred.

The raw materials preferably include the lactulose. This may be a commercially available product, and examples of the commercially available product include the milk oligosaccharides MLC-90, MLP-95, and MLS-95 (manufactured by Morinaga Milk Industry Co., Ltd.). The milk oligosaccharide preferably contains lactulose at preferably 30% by mass or more, more preferably 40% by mass or more, still more preferably 50% by mass or more.

1-3-2-3. Example of Method of Producing Fermented Food or Beverage of Present Technique

The method of producing a fermented food or beverage for lowering of the blood pressure and/or reduction of neutral fat is described below. More specifically, an example is described for a case where a casein hydrolysate and an indigestible dextrin are used as unfermented raw materials. However, the method of producing a fermented food or beverage of the present technique is not limited thereto.

In the production method of the present technique, a liquid milk preparation containing a casein hydrolysate and an indigestible dextrin as raw materials of a fermentation milk may be sterilized, and a fermentative bacterium/bacteria may be added to the sterilized liquid milk preparation, followed by allowing fermentation until the pH becomes 5.0 or less.

Regarding the fermentative bacterium/bacteria, the fermentation is preferably carried out by addition of a lactic acid bacterial starter and/or a bacterium/bacteria belonging to the genus *Bifidobacterium.*

As raw materials of the fermented milk used in the present technique, common raw materials of fermented milks may be used as long as the casein hydrolysate and the indigestible dextrin are blended.

The common raw materials of fermented milks are not limited as long as they are milk-derived raw materials that enable production of a fermented food or beverage by fermentation using a fermentative bacterium/bacteria. Examples of the raw materials include milk, and its fractionated products and processed products (for example, cow's milk, skim milk, fresh cream, butter, whole milk powder, and non-fat dry milk; and products prepared by mixing, dissolving or suspending these materials in water). The animal from which the milk is derived is not limited. The milk is preferably derived from cows.

Other examples of raw materials of the fermented milk, which may be blended when necessary, include arbitrary components such as sweeteners including sucrose; pectin; fruits; fruit juices; agar; gelatin; oils and fats; flavoring agents; coloring agents; stabilizers; and reducing agents.

Before fermentation, the raw materials of the fermented milk may be subjected to sterilization, homogenization, cooling, and/or the like according to conventional methods.

The fermentation may be carried out by adding a lactic acid bacterial starter and/or a fermentative bacterium/bacteria including a bacterium belonging to the genus *Bifidobacterium,* to the raw materials of the fermented milk (preferably a liquid milk preparation). Among these fermentative bacteria, an arbitrary bacterium/bacteria may be inoculated a plurality of times.

The "lactic acid bacterial starter" inoculated to the raw materials of the fermented milk may be either a single bacterial strain or a combination of a plurality of bacterial strains.

The "lactic acid bacterial starter" in the present technique is not limited as long as it is for use in production of foods and beverages. In cases where the fermented food or beverage is yogurt, examples of the "lactic acid bacterial starter" include *Lactobacillus bulgaricus, Lactobacillus delbrueckii* subsp. *bulgaricus,* and *Streptococcus thermophilus.* Among these lactic acid bacteria, either a single bacterial strain or a combination of a plurality of bacterial strains may be used.

The amount of the fermentative bacterium/bacteria in the present technique inoculated to the raw materials of the fermented milk is not limited.

The amount of the lactic acid bacterium/bacteria and the bacterium/bacteria belonging to the genus *Bifidobacterium* in the present technique is preferably $10^4$ to $10^9$ CFU/mL milk raw materials, more preferably $10^6$ to $10^8$ CFU/mL milk raw materials.

The amount of the bacterium/bacteria belonging to the genus *Bifidobacterium* is preferably $10^5$ to $10^9$ CFU/mL milk raw materials, more preferably $10^7$ to $10^8$ CFU/mL milk raw materials.

The bacterium/bacteria belonging to the genus *Bifidobacterium* and/or the other lactic acid bacterium/bacteria to be inoculated to the fermented-milk raw materials is/are preferably preliminarily subjected to seed culture or preculture in another medium.

The medium used for the main culture is not limited as long as it is a medium suitable for culture of bacteria belonging to the genus *Bifidobacterium* and/or other lactic acid bacteria. Examples of the medium include media containing reduced non-fat dry milk.

The concentration of the reduced non-fat dry milk is preferably 3% (W/W) or more, especially preferably 8% (W/W) or more. The medium used for the seed culture or preculture may contain a growth-promoting substance such as a yeast extract, a reducing agent such as L-cysteine, or the like.

In particular, in cases where a bacterium belonging to the genus *Bifidobacterium* is used, its growth ability in a medium containing reduced non-fat dry milk is low, so that a medium containing a growth-promoting substance, for example, 0.1 to 1% (W/W) yeast extract, is preferably used. The sterilization conditions for the medium are the same as described above.

Regarding the fermentation conditions such as the culture temperature and the culture period in the method of producing a fermented food or beverage of the present technique, the same conditions as in production of a fermented food or beverage from ordinary milk raw materials may be employed. For example, the culture temperature is preferably 30°C to 42°C, more preferably 36°C to 40°C. The culture period may be appropriately set in accordance with the type of the fermented food or beverage to be produced. The culture period is usually preferably 4 to 18 hours.

The fermented food or beverage obtained by the method of producing a fermented food or beverage of the present technique may be processed as appropriate in the same manner as fermented foods and beverages from ordinary milk raw materials. For example, the fermented food or beverage after the fermentation may be provided as it is as a food, or may be processed into a liquid form by homogenization. The fermented food or beverage may also contain a sweetener such as sucrose; pectin; fruit; fruit juice; agar; gelatin; oil or fat; flavoring agent; coloring agent; stabilizer; reducing agent; or the like. The fermented food or beverage may be filled into a container as appropriate.

Since the fermented food or beverage obtained as described above contains the casein hydrolysate and the indigestible dextrin, it can favorably produce the effect of the present technique.

From the viewpoint of also improving the taste of the fermented food or beverage containing the casein hydrolysate and the indigestible dextrin of the present technique, sucralose, fructose, rare sugar, lactulose, and/or the like is/are preferably further used.

As described later in <2. Second Embodiment of Present Technique: Method of Producing Fermented Milk Containing Milk Protein Hydrolysate and/or Indigestible Dextrin>, since the fermented food or beverage of the present technique uses the casein hydrolysate and the indigestible dextrin as raw materials, it tends to have bitterness or an unpleasant odor due to these peptides, and an unpleasant odor due to starch. In such a case, (1) sucralose; and/or (2) one or more monosaccharides and/or rare sugars selected from the group consisting of fructose, psicose, and allose; is/are preferably used. The use of the sucralose, rare sugar, and/or the like enables masking of the unpleasant odor and the like of the fermented food or beverage of the present technique, and also enables improvement of the taste of the fermented food or beverage containing the casein hydrolysate and the indigestible dextrin, while maintaining the flavor specific to the fermented food or beverage as much as possible. Further, in the fermented food or beverage of the present technique, use of (3) lactulose or milk oligosaccharide enables suppression of the unpleasant odor and the like and achievement of a better milk taste.

1-3-2-4. Food or Beverage with Functional Claims

The food, beverage, or the like defined in the present technique may be provided or sold as a food or beverage whose label indicates a specific intended use (in particular, health use) or function.

The act of "labeling" includes any act for informing the intended use to consumers. Any expression that allows the consumers to assume or infer the intended use corresponds to the act of "labeling" in the present technique irrespective of, for example, the purpose of the label, the content of the label, and to what object or medium the label is applied.

The "labeling" is preferably carried out with an expression with which the consumers are capable of directly recognizing the intended use. More specifically, examples of the act of labeling include acts of transfer of, delivery of, displaying for transfer or delivery of, or import of, a food- or beverage-related product or packaging of the product on which the intended use is described, and acts of describing the intended use in an advertisement, price list, or transaction document related to the product, and displaying or distributing it, or describing the intended use in information including such a content and providing the information by an electromagnetic method (internet or the like).

On the other hand, regarding the content of the label, the label is preferably a label approved by the government or the like (for example, a label with approval based on a system(s) established by the government, which label is applied in a mode in accordance with the approval). It is preferred to apply such a content of the label to packaging, containers, catalogs, pamphlets, advertisement materials in sales locations such as POPs, or other documents.

Examples of the "label" also include labels for health foods, functional foods, patient foods, enteral foods, special purpose foods, foods with health claims, foods for specified health uses, foods with function claims, foods with nutrient function claims, quasi drugs, and the like. Among these, particular examples of the "label" include labels approved by the Consumer Affairs Agency, such as labels approved based on the food for specified health uses system, food with function claims system, or a system similar thereto. More specific examples of the "label" include labels for foods for specified health uses, labels for conditional foods for specified health uses, labels for foods with function claims, labels indicating that a structure or function of the body is affected, and labels indicating reduction of disease risk. Among these, typical examples of the "label" include labels for foods for specified health uses (especially labels indicating health uses) prescribed by the enforcement regulations of Health Promotion Law (Japan Ministry of Health, Labor and Welfare, Ministerial Ordinance No. 86, Apr. 30, 2003), labels for foods with function claims prescribed by the Food Labeling Act (Act No. 70, 2013), and labels similar thereto.

The content of the casein hydrolysate used for the food, beverage, or the like according to the present technique may be arbitrarily set in accordance with the age, symptoms, and the like as long as the effect of the present technique is not deteriorated. In particular, in the present technique, the content of the casein hydrolysate may be set to 0.001 to 1 % by mass for the final composition such as a food or beverage.

In the present technique, the amount of the casein hydrolysate used for the subject is preferably 0.0001 to 1 g/kg body weight/day, more preferably 0.0005 to 0.5 g/kg body weight/day, still more preferably 0.001 to 0.1 g/kg body weight/day.

The content of the indigestible dextrin used for the food, beverage, or the like according to the present technique may also be arbitrarily set in accordance with the age, symptoms, and the like as long as the effect of the present technique is not deteriorated. In the present technique, for example, in cases where the amount of the indigestible dextrin for each meal is about 5 g, the content of the indigestible dextrin may be set to 1 to 10% by mass for the final composition such as a food or beverage.

In the present technique, the amount of the indigestible dextrin used for the subject is preferably 0.001 to 10 g/kg body weight/day, more preferably 0.005 to 5 g/kg body weight/day, still more preferably 0.01 to 1 g/kg body weight/day.

Thus, in the composition provided in the form of a food, beverage, or the like, the content of each of the indigestible dextrin and the casein hydrolysate is not limited. From the viewpoint of better production of the effect of the present technique, the mass ratio between their contents or amounts used is preferably indigestible dextrin : casein hydrolysate = 10:0.001 to 1:1, more preferably indigestible dextrin : casein hydrolysate = 1:0.001 to 10:1.

The content or the amount of each of the tripeptide MKP and the indigestible dextrin used in the present technique is not limited. From the viewpoint of better production of the effect of the present technique, the content or the amount of the indigestible dextrin used with respect to 1 part by mass of the tripeptide MKP is, regarding the lower limit, preferably 5,000 parts by mass or more, more preferably 10,000 parts by mass or more, and, regarding the upper limit, preferably 100,000 parts by mass or less, more preferably 80,000 parts by mass or less. Within this range, the content or the amount of the indigestible dextrin is preferably 15,000 to 50,000 parts by mass.

<1-3-3. Feed>

For utilization of the excellent effect, the present technique may be used as an effective component in an animal feed to be used for lowering of the blood pressure and/or reduction of neutral fat, and/or for prevention, amelioration, and/or treatment of hypertension and/or dyslipidemia (hyperlipidemia). The present technique may be prepared by addition to a known feed, or a novel feed may be produced by mixing with a feed raw material(s).

Examples of raw materials of the feed include cereals such as corn, wheat, barley, and rye; brans such as wheat bran, barley bran, rice bran, and defatted rice bran; production meals such as corn gluten meal and corn germ meal; animal-derived feeds such as non-fat dry milk, whey, fish flour, and powdered bone; yeasts such as brewer's yeast; mineral feeds such as calcium phosphate and calcium carbonate; oils and fats; amino acids; and sugars. Examples of the form of the feed include feeds for pet animals (pet foods), livestock feeds, and fish feeds.

The amount of the casein hydrolysate used for the feed according to the present technique may be arbitrarily set in accordance with the body weight, symptoms, and the like as long as the effect of the present technique is not deteriorated. In particular, in the present technique, the amount of the casein hydrolysate may be set to 0.001 to 1% by mass for the final composition of the feed.

In the present technique, the amount of the casein hydrolysate used is preferably 0.0001 to 1 g/kg body weight/day, more preferably 0.0005 to 0.5 g/kg body weight/day, still more preferably 0.0001 to 0.1 g/kg body weight/day.

The content of the indigestible dextrin used for the feed according to the present technique may also be arbitrarily set in accordance with the body weight, symptoms, and the like as long as the effect of the present technique is not deteriorated. In the present technique, for example, in cases where the amount of the indigestible dextrin for each meal is about 5 g, the content of the indigestible dextrin may be set to 1 to 10% by mass for the final composition of the feed.

In the present technique, the amount of the indigestible dextrin used is preferably 0.001 to 10 g/kg body weight/day, more preferably 0.005 to 5 g/kg body weight/day, still more preferably 0.01 to 1 g/kg body weight/day.

Thus, in the composition provided in the form of a feed, the content or the amount of each of the indigestible dextrin and the casein hydrolysate used is not limited. From the viewpoint of better production of the effect of the present technique, the mass ratio between their contents or amounts used is preferably indigestible dextrin : casein hydrolysate = 10:0.001 to 1:1, more preferably indigestible dextrin : casein hydrolysate = 1:0.001 to 10:1.

The content or the amount of each of the tripeptide MKP and the indigestible dextrin used in the present technique is not limited. From the viewpoint of better production of the effect of the present technique, the content or the amount of the indigestible dextrin used with respect to 1 part by mass of the tripeptide MKP is, regarding the lower limit, preferably 5,000 parts by mass or more, more preferably 10,000 parts by mass or more, and, regarding the upper limit, preferably 100,000 parts by mass or less, more preferably 80,000 parts by mass or less. Within this range, the content or the amount of the indigestible dextrin is preferably 15,000 to 50,000 parts by mass.

<2. Second Embodiment of Present Technique: Method of Producing Fermented Milk Containing Milk Protein Hydrolysate and/or Indigestible Dextrin>

As another aspect of the present technique (which may also be referred to as "the second embodiment of the present technique" or "the second embodiment"), a method of producing a fermented milk containing a milk protein hydrolysate and/or an indigestible dextrin, a fermented milk, and improvement of the fermented-milk taste are provided.

Fermented milks such as yogurt have become popular as healthy foods and beverages consumed on a daily basis since, for example, they have good tastes derived from lactic acid and flavor components produced by fermentation, and intestinal regulation actions due to the presence of fermentative bacteria such as lactic acid bacteria and bifidobacteria.

Patients with lifestyle-related diseases such as obesity, diabetes, dyslipidemia, hypertension, and hyperuricemia, and high-risk groups of these diseases (that is, borderline patients), have been increasing mainly in developed countries. Lifestyle-related diseases also occur in non-human animals because of keeping the animals indoors and/or feeding the animals pet food.

One possible approach to improvement of the lifestyle is alteration of the content of meals. In relation to the alteration, there is a report suggesting improvement of lifestyle-related diseases by ingestion of a fermented milk such as yogurt. Therefore, ingestion of a fermented milk has been recommended in some cases.

Further, methods of producing fermented milks that enhance the functionality as a countermeasure against lifestyle-related diseases have been studied. For example, Reference 11 (JP-A-2017-184682) proposes a method of producing a fermented milk, which method enables production of a low phosphorus fermented milk having a pleasant taste.

For enhancement of the functionality of a fermented milk, the present inventors studied a method of producing a fermented milk containing a milk protein hydrolysate and/or an indigestible dextrin. It has been reported that casein hydrolysates and indigestible dextrins have effects that enable, for example, prevention or amelioration of lifestyle-related diseases, such as blood glucose elevation-suppressing actions.

However, as described later in the Examples, a fermented milk using a milk protein hydrolysate as a fermented-milk raw material produced unpleasant bitterness derived from the milk protein hydrolysate. Further, a fermented milk using an indigestible dextrin as a fermented-milk raw material produced an unpleasant starchy odor derived from the indigestible dextrin. Further, a fermented milk using a milk protein hydrolysate and an indigestible dextrin as fermented-milk raw materials produced a stronger unpleasant odor, and had a deteriorated fermented-milk taste.

One possible solution therefor may be use of a compound having a masking effect on the bitterness derived from peptides, the odor specific to peptides (hereinafter referred to as peptide odor), and the starchy odor derived from dextrin. However, there is a concern that the masking effect may deteriorate the important flavor and taste of the fermented milk.

In view of this, another aspect of the present technique may be providing a fermented milk in which, even in cases where a milk protein hydrolysate and/or an indigestible dextrin is/are used as a fermented-milk raw material(s), the fermented milk-specific flavor and/or taste can be maintained as much as possible while favorably masking the bitterness, starchy odor, and unpleasant odor derived from the milk protein hydrolysate and/or the indigestible dextrin. This may be the second main object, which is separate from the above-described present technique <1. Composition Having Blood pressure-Lowering Action and/or Neutral Fat-Reducing Action According to Present Technique>. However, the <1. Composition Having Blood pressure-Lowering Action and/or Neutral Fat-Reducing Action According to Present Technique> is not limited by the second main object.

In relation to the other aspect of the present technique, the present disclosers used sucralose for a fermented milk containing a casein hydrolysate and/or an indigestible dextrin as described later in the Examples. As a result, the flavor of the fermented milk deteriorated, and the masking effect on the unpleasant odor and the starchy odor was insufficient. Further, when the masking was carried out using a rare sugar-containing syrup, the flavor of the fermented milk deteriorated, and the masking effect on the bitterness and the unpleasant odor was insufficient. Thus, as a result, the masking effect caused deterioration of the important flavor and taste of the fermented milk as expected.

However, as a result of intensive study on the other aspect of the present technique, the present disclosers discovered that, even in cases where a milk protein hydrolysate and/or an indigestible dextrin is/are used as a raw material(s), use of sucralose, and one or more monosaccharides selected from the group consisting of fructose, psicose, and allose, in combination therewith enables production of a fermented milk maintaining the fermented milk-specific flavor as much as possible while masking the bitterness and the unpleasant odor of peptides, and the unpleasant odor of starch.

Further, regarding the other aspect of the present technique, the present disclosers also discovered that, even in cases where a milk protein hydrolysate and/or an indigestible dextrin is/are used as a raw material(s), use of a lactulose-containing syrup as a raw material in addition to the sucralose and the rare sugar enables production of a fermented milk having an even better milk taste.

The raw materials in the second embodiment are raw materials that may be used in any step in the method of producing a fermented milk, and may be used as pre-fermentation raw materials and/or post-fermentation raw materials.

Thus, the present disclosers completed the second embodiment. More specifically, the second embodiment is as described in the following [1] to [10].

[1] A method of producing a fermented milk containing (A) a milk protein hydrolysate and/or (B) an indigestible dextrin, the method including the steps:
including (C) sucralose and (D) one or more monosaccharides such as fructose, psicose, and allose.

[2] The method of producing a fermented milk according to [1], wherein the fermented milk further contains (E) lactulose.

[3] The method of producing a fermented milk according to [1] or [2], wherein the milk protein hydrolysate is a casein hydrolysate.

[4] The method of producing a fermented milk according to any one of [1] to [3], wherein the milk protein hydrolysate contains a peptide composed of Met-Lys-Pro.

[5] The method of producing a fermented milk according to any one of [1] to [4], wherein the fermented milk has an improved taste.

[6] A fermented milk containing the following (A) and/or (B), and also containing the following (C) and (D):

(A) milk protein hydrolysate;
(B) indigestible dextrin;
(C) sucralose;
(D) one or more monosaccharides such as fructose, psicose, and allose.

[7] The fermented milk according to [6], further containing (E) lactulose.

[8] The fermented milk according to [7], which is a fermented milk having an improved taste.

[9] A method of improving the taste of a fermented milk containing (A) a milk protein hydrolysate and/or (B) an indigestible dextrin, the method including the steps:

using (C) sucralose and (D) one or more monosaccharides such as fructose, psicose, and allose.

[10] The method of improving the taste of a fermented milk according to [9], further using lactulose.

The second embodiment can provide a fermented milk in which, even in cases where a milk protein hydrolysate and/or an indigestible dextrin is/are used as a fermented-milk raw material(s), the fermented milk-specific flavor can be maintained as much as possible while masking the bitterness, starchy odor, and unpleasant odor derived from the milk protein hydrolysate and/or the indigestible dextrin. The effects described herein are not necessarily limited, and may be any of the effects described for the present technique.

Preferred embodiments for carrying out the second embodiment are described below.

<2-1. Method of Producing Fermented Milk Containing Milk Protein Hydrolysate and/or Indigestible Dextrin According to Second Embodiment of Present Technique>

The second embodiment is a method of producing a fermented milk containing (A) a milk protein hydrolysate and/or (B) an indigestible dextrin, the method including the steps of:

using (C) sucralose and (D) one or more monosaccharides such as fructose, psicose, and allose.

The fermented milk preferably further contains (E) lactulose, or uses lactulose.

The (A) to (E) may be added or used as fermented-milk raw materials in any step of the production process of the fermented milk. They may be used before the fermentation step and/or after the fermentation step.

By the combination with (C) sucralose and (D) one or more monosaccharides such as fructose, psicose, and allose, the taste of the fermented milk containing (A) the milk protein hydrolysate and/or (B) the indigestible dextrin can be improved while the masking effect on (A) and/or (B) can be obtained. By further using (E) lactulose, the milk taste of the fermented milk containing (A) and/or (B) can be further improved, resulting in an even better taste of the whole fermented milk.

The "improvement of the taste of a fermented milk" in the present description means that the fermented milk-specific flavor is at least not deteriorated while the bitterness, starchy odor, and unpleasant odor are at least masked, compared to fermented milks which do not contain (C) to (E), but which contain (A) a milk protein hydrolysate and/or (B) an indigestible dextrin.

In general, in cases where (A) a milk protein hydrolysate and (B) an indigestible dextrin are used in combination, some humans feel not only the bitterness and the starchy odor, but also an animal odor. The components which humans generally feel as the fermented milk-specific flavor have not been sufficiently clarified. As flavors of components of fermented milks and flavors derived from fermentation by lactic acid bacteria, diacetyl and acetaldehyde are known. Examples of the milk taste include the richness of the milk, the feeling of milk fat, and the taste of a cow's milk.

Since the fermented milk produced by the production method of the second embodiment contains the milk protein hydrolysate and/or the indigestible dextrin, various effects are produced by their individual use or combined use.

Compared to fermented milks containing (A) a milk protein hydrolysate and/or (B) an indigestible dextrin, the fermented milk produced by the production method of the second embodiment has an improved fermented-milk taste and a better taste since the fermented milk-specific flavor can be maintained while the bitterness, starchy odor, and unpleasant odor due to the (A) and/or (B) can be masked. In the second embodiment, the improvement of the overall taste of the fermented milk can also be said to be an excellent effect.

Thus, since the fermented milk of the second embodiment has a good taste, individuals expecting the effect from the (A) and/or (B) can easily ingest the fermented milk of the second embodiment on a daily basis. Therefore, the fermented milk of the second embodiment is effective also from the viewpoint of easily allowing continuous production of the effect. With the fermented milk of the second embodiment, an effect produced by any of, or a combination of, the (C) to (E) used as fermented-milk raw materials can also be expected.

Expecting such an effect of the second embodiment, the second embodiment may be employed for the <Blood pressure-Lowering and/or Neutral Fat-Reducing Composition According to Present Technique>.

<2-2. Method of Producing Fermented Milk of Second Embodiment>

<2-2-1. Fermented-Milk Raw Materials in Second Embodiment>

As fermented-milk raw materials used in the production method of the second embodiment, (A) the milk protein hydrolysate and/or (B) the indigestible dextrin are used, and further, (C) sucralose and (D) one or more monosaccharides such as fructose, psicose, and allose (which may be hereinafter also referred to as "(D) monosaccharide(s)") are used. Further, (E) lactulose is preferably used as a fermented-milk raw material of the present technique.

Regarding the (A) to (E) used in the second embodiment, each of these may be added, as a raw material used for the method of producing a fermented milk, in any step of the production process of the fermented milk. They may be added either before the fermentation or after the fermentation as long as they are contained in the fermented milk, which is the final product. In the second embodiment, the components (A) to (E) are used as raw materials of the fermented milk. However, they do not necessarily need to be fermented, and may be added after the fermentation.

In one example of the second embodiment, one or more, or all, of the (A) to (D) that have not been added before the fermentation may be added to the fermented milk after the fermentation. Further, in one example of the present technique, one or more of the (A) to (D) may be added both before the fermentation and after the fermentation, to include the (A) to (D) in the final product. The (E) is preferably added before the fermentation and/or after the fermentation, to include it in the final product.

<2-2-2. Components (A) to (E) as Fermented-Milk Raw Materials>

The components (A) to (E) used as fermented-milk raw materials in the second embodiment are described below.

<2-2-2-1. (A) Milk Protein Hydrolysate>

The (A) milk protein hydrolysate used in the second embodiment is not limited, and is preferably a hydrolysate of a milk protein derived from cow's milk or from a milk product such as non-fat dry milk.

Examples of the hydrolysis include acid hydrolysis, alkaline hydrolysis, and enzyme hydrolysis. Among these, enzyme hydrolysis is preferred from the viewpoint of easier preparation of various desired peptide components. The enzyme hydrolysis may be carried out according to the later-mentioned enzyme hydrolysis conditions.

Examples of the milk-derived protein include casein protein and whey protein. One or more of these may be selected.

In general, casein protein can be classified into the following three types: $\alpha$-casein, $\beta$-casein, and $\kappa$-casein. In general, whey protein is also called milk serum protein or soluble protein.

In general, whey protein can be classified into serum albumin, $\beta$-lactalbumin, $\alpha$-lactalbumin, immunoglobulin, proteose-peptone, and the like.

In the present technique, a hydrolysate of casein protein (which may be hereinafter also referred to as "casein hydrolysate") is preferred from the viewpoint of favorable improvement of the fermented-milk taste in the present technique, and from the viewpoint of effective utilization of the effect expected with the casein hydrolysate.

<2-2-2-1-1. Casein Hydrolysate>

In the following description, concerning the casein hydrolysate used in the second embodiment, the method of producing the casein hydrolysate used in the second embodiment, and the tripeptide MKP, description of the constitutions overlapping with the <1-1-1. Method of Producing Casein Hydrolysate> is omitted as appropriate.

The casein hydrolysate used in the present technique is obtained by hydrolysis of casein protein derived from milk, and contains various degraded components derived from the casein protein.

The casein hydrolysate preferably satisfies at least any of the following (a1) to (a4). More preferably, two or more of the conditions (a1), (a2), (a3), and (a4) are satisfied.

(a1) The average molecular weight of the casein hydrolysate is 1,200 daltons or less;

(a2) the degradation rate of the casein hydrolysate is 20 to 30%;

(a3) the mass ratio of free amino acids in the total mass of all amino acids contained in the casein hydrolysate is 10% by mass or less; and

(a4) a peptide composed of Met-Lys-Pro is contained.

From the viewpoint of favorable production of the effect of the second embodiment, the casein hydrolysate preferably contains at least a peptide composed of Met-Lys-Pro (SEQ ID NO:1) (hereinafter also referred to as "tripeptide MKP").

Preferably, the casein hydrolysate contains the tripeptide MKP, and satisfies one or more conditions selected from the (al), (a2), and (a3). The casein hydrolysate more preferably satisfies all of the conditions (a1) to (a4).

The content of the tripeptide MKP in the casein hydrolysate is not limited. Regarding the lower limit of the content of the tripeptide MKP, from the viewpoint of better production of the effect of the second embodiment, the content is preferably 0.005% by mass or more, more preferably 0.01% by mass or more. Regarding the upper limit of the content, from the viewpoint of the production efficiency of the degradation product in the second embodiment, the content is preferably 0.2% by mass or less, more preferably 0.1% by mass or less. The range of this value is still more preferably 0.01 to 0.1% by mass.

The casein hydrolysate used in the second embodiment is preferably prepared such that it has an average molecular weight of preferably 1,200 daltons or less, more preferably 1,000 daltons or less, still more preferably 800 daltons or less, still more preferably 450 daltons or less, from the viewpoint of better production of the effect of the second embodiment. The preparation is preferably carried out to achieve an average molecular weight of more preferably 250 to 450 daltons, still more preferably 360 to 390 daltons.

The casein hydrolysate used in the second embodiment is preferably prepared such that it has a degradation rate of preferably 10% or more regarding the lower limit, and preferably 40% or less regarding the upper limit; more preferably prepared such that it has a degradation rate of 20 to 30%, from the viewpoint of better production of the effect of the second embodiment.

In the second embodiment, reaction conditions such as the reaction temperature and the reaction duration are preferably determined so as to set the degree of hydrolysis such that the casein hydrolysate contained in the filtrate after the removal of the insoluble matter produced due to the hydrolysis has an average molecular weight within a desired range, and/or such that the casein hydrolysate has a degradation rate within a desired range.

The casein hydrolysate used in the second embodiment is more preferably prepared such that the ratio of the total mass of free amino acids in the total mass of all amino acids contained therein is preferably 15% by mass or less, more preferably 10% by mass or less, from the viewpoint of better production of the effect of the second embodiment.

The casein hydrolysate used in the second embodiment is preferably prepared such that the ratio of the tripeptide MKP contained therein is preferably 0.001 to 1% by mass, more preferably 0.005 to 0.5% by mass, still more preferably 0.01 to 0.1% by mass, from the viewpoint of the effect and the production efficiency of the second embodiment.

In the second embodiment, the ratio of the total mass of free amino acids or the ratio of the tripeptide MKP may be adjusted such that each desired ratio can be achieved by, for example, selecting the type of the enzyme for the hydrolysis of casein, the amount of the enzyme added, the reaction time, and/or purification conditions (membrane separation, resin adsorption separation, or the like) after the hydrolysis.

<Degradation Rate of Amino Acids>, <Method of Calculating Average Molecular Weight>, <Method of Calculating Free Amino Acid Ratio>, and <Measurement of Tripeptide MKP Content> in the casein hydrolysate in the second embodiment are the same as in the <1. Composition Having Blood pressure-Lowering Action and/or Neutral Fat-Reducing Action According to Present Technique>.

<2-2-2-2. (B) Indigestible Dextrin>

The indigestible dextrin used in the second embodiment is the same as the <1-2. Indigestible Dextrin>.

<2-2-2-3. (C) Sucralose>

Sucralose (another name: 4,1',6'-trichlorogalactosucrose), which is used in the second embodiment, is also called high-intensity sweetener. It is known as an artificial sweetener having higher sweetness than sucrose and capable of giving sweetness to foods and beverages even when it is used in a very small amount. The sucralose can be obtained as a commercially available product. The sucralose can be measured by quantitative analysis of sugar by high-performance liquid chromatography (HPLC).

<2-2-2-4. (D) One or More Monosaccharides Selected from Group Consisting of Fructose, Psicose, and Allose>

The one or more monosaccharides such as fructose, psicose, and allose used in the second embodiment are known as sweeteners. Fructose is also called fruit sugar, and it is preferably D-fructose. Psicose and allose are rare sugars, and D-psicose and D-allose are preferred. They are known to suppress blood glucose elevation, and to reduce body fat.

As the one or more monosaccharides such as fructose, psicose, and allose used in the second embodiment, a rare sugar-containing syrup is preferably used from the viewpoint of easier achievement of the object of the second embodiment, and from the viewpoint of availability.

Since the rare sugars psicose and allose are present only in small amounts in nature, for example, a rare sugar-containing syrup produced by a production method described in Reference 12 (WO 2010/113785) (hereinafter referred to as "rare sugar-containing isomerized sugar") may be used, or a commercially available rare sugar-containing syrup (Rare Sugar Sweet, manufactured by Matsutani Chemical Industry Co., Ltd.) may be used. Fructose, glucose, psicose, and allose can be measured by quantitative analysis of sugar by high-performance liquid chromatography (HPLC).

The rare sugar-containing syrup preferably contains 0.5 to 17% by mass D-psicose, 0.2 to 10% by mass D-allose, 10 to 40% by mass (preferably 25 to 35% by mass) D-fructose, and 15 to 55% by mass (preferably 35 to 50% by mass) D-glucose. The rare sugar-containing syrup may also contain other rare sugars.

The rare sugar-containing syrup used in the second embodiment is a syrup containing an isomerized sugar and rare sugar, and preferably contains 1 to 150 parts by mass of the rare sugar with respect to 100 parts by mass of the isomerized sugar. The isomerized sugar is a liquid sugar containing glucose and fructose as major components.

The rare sugar-containing syrup is obtained by isomerizing one or more of D-glucose, D-fructose, and isomerized sugar with an alkali at 0.005 mol/L or more. The amounts of sugars other than D-glucose and D-fructose is preferably less than 60% by mass.

In general, an isomerized sugar is produced by saccharifying starch to prepare a saccharified solution, and then treating the saccharified solution with glucose isomerase. The saccharified solution is produced, for example, as a glucose solution prepared by hydrolyzing starch with an enzyme to produce dextrin, and then further hydrolyzing the dextrin with another enzyme. The isomerization reaction from glucose to fructose by glucose isomerase is an equilibrium reaction, and the glucose:fructose mass ratio in the isomerized sugar is usually about 58:42. In some cases, the mass ratio is usually set to about 45:55 by adding fructose in order to increase the sweetness. Thus, in an industrial isomerized sugar, the glucose:fructose mass ratio is usually about 45 to 58 : 55 to 42.

The rare sugar-containing syrup used in the second embodiment can be produced by subjecting an isomerized sugar liquid of a raw material sugar liquid to treatment in a system in which one or more of a basic ion-exchange resin, alkali, and calcium salt are present, to cause an isomerization reaction. By this, a syrup containing D-psicose, D-allose, D-fructose, and D-glucose can be obtained, and the syrup contains 0.5 to 17% by mass D-psicose and 0.2 to 10% by mass D-allose.

More preferably, all of, or at least one of, the following is/are carried out. Still more preferably, all of these are carried out.

(1) For the treatment in the system in which the basic ion-exchange resin is present, the isomerized sugar liquid of the raw material sugar liquid is preliminarily prepared as an alkaline solution, and an isomerization reaction under alkaline conditions is carried out on the resin, to increase the yield of D-psicose and D-allose.
(2) For the treatment in the system in which the alkali is present, the concentration of the alkali in the raw material sugar liquid is maintained at 0.005 to 2 mol/L.
(3) For the treatment in the system in which the calcium salt is present, the calcium salt concentration in the raw material sugar liquid is maintained at 0.005 to 6 mol/L.

Further, after the treatment of the isomerized sugar liquid in the system in which one or more of the basic ion-exchange resin, alkali, and calcium salt are present, the liquid is preferably passed through an acidic ion-exchange resin and/or a mixed resin, to perform the reaction, neutralization, and desalting in a series of steps.

Although the rare sugar-containing syrup in the second embodiment is not limited by the following production example, a more specific example of the rare sugar-containing syrup is as follows. A commercially available isomerized sugar (fructose, 42%) is diluted with 0.1 M NaOH solution to 30% (w/v), and the resulting solution is transferred to a prepared strongly basic ion-exchange resin (resin: Amberlite IRA900J[OH]) at a temperature of 60°C and an SV (space velocity: flow volume (1) / time (h) / resin volume (1)) of 1. Subsequently, according to a conventional method, the eluted sugar liquid is purified and concentrated with an ion-exchange resin, to obtain an isomerized sugar containing a rare sugar. This results in about 44% by mass D-glucose, about 32% by mass D-fructose, about 6% by mass D-psicose, about 1.5% by mass D-allose, and about 6% by mass other rare sugar monosaccharides. The mass ratios of the monosaccharides can be determined by performing analysis by HPLC (detector, RI; column, MCI GEL CK 08EC, manufactured by Mitsubishi Kasei Corporation), and then performing calculation based on the peak area.

<2-2-2-5. (E) Lactulose>

The lactulose preferably used in the second embodiment is obtained using lactose as a raw material. It is a disaccharide in which galactose is bound to fructose, and is an oligosaccharide contained as a major component in a lactulose-containing syrup (also called "milk oligosaccharide"). As the lactulose used in the second embodiment, a milk oligosaccharide is preferably used from the viewpoint of easier achievement of the object of the second embodiment, and from the viewpoint of availability.

The lactulose may be produced by, for example, a method disclosed in Reference 13 (JP-A-3-169888) or Reference 14 (JP-A-6-228179), or may be a commercially available product. The lactulose can be measured by quantitative analysis of sugar by high-performance liquid chromatography (HPLC).

A commercially available milk oligosaccharide such as the lactulose "Milk Oligosaccharide MLC-97" (manufactured by Morinaga Milk Industry Co., Ltd.), "Milk Oligosaccharide MLP-95" (manufactured by Morinaga Milk Industry Co., Ltd.), or "Milk Oligosaccharide MLS-95" (manufactured by Morinaga Milk Industry Co., Ltd.) may also be used.

The milk oligosaccharide may contain a sugar derived from lactose other than lactulose, for example, galactooligosaccharide. From the viewpoint of better production of the effect of the second embodiment, the milk oligosaccharide contains lactulose at preferably 30% by mass or more, more preferably 40% by mass or more, still more preferably 50% by mass or more.

<2-2-2-6. Amounts of Components (A) to (E) in Fermented-Milk Final Product>

In the second embodiment, the amount of each of (A) to (E) may be adjusted in the production process of the fermented milk such that the content in the final product of the fermented milk becomes as follows. For example, by adding each of the components (A) to (E) as appropriate after the fermentation, the amount of each of (A) to (E) in the final product of the fermented milk can be adjusted.

In the second embodiment, the amount of (A) the milk protein hydrolysate in the final product is not limited. Regarding the lower limit, the amount is preferably 0.1% by mass or more, more preferably 0.25% by mass or more, still more preferably, from the viewpoint of production of the effect of the second embodiment, 0.45% by mass or more. Regarding the upper limit, the amount is preferably 2% by mass or less, more preferably 1.5% by mass or less, still more preferably, from the viewpoint of improving the taste, 1% by mass or less. In the final product, the amount of (A) the milk protein hydrolysate is more preferably 0.1 to 1.0% by mass, still more preferably 0.3 to 0.8% by mass. An amount within this range is preferred from the viewpoint of production of the effect of the second embodiment.

In the second embodiment, the final product preferably contains (a4) the tripeptide MKP. The content of (a4) the tripeptide MKP in the final product is not limited. Regarding the lower limit, the amount is preferably 0.00002% by mass or more, more preferably 0.00009% by mass or more, still more preferably, from the viewpoint of production of the effect of the second embodiment by this peptide, 0.0001% by mass or more. Regarding the upper limit, the amount is preferably 0.001% by mass or less, more preferably 0.0008% by mass or less, still more preferably 0.0005% by mass or less. The amount of (a4) the tripeptide MKP in the final product is more preferably 0.00002 to 0.001% by mass, still more preferably 0.00009 to 0.0008% by mass, still more preferably 0.0001 to 0.0005% by mass. An amount within this range is preferred from the viewpoint of production of the effect of the second embodiment.

In the second embodiment, the content of (B) the indigestible dextrin in the final product is not limited. Regarding the lower limit, the amount is preferably 0.5% by mass or more, more preferably 1% by mass or more, still more preferably, from the viewpoint of production of the effect of the second embodiment by the indigestible dextrin, 4% by mass or more. Regarding the upper limit, the amount is preferably 15% by mass or less, more preferably 10% by mass or less, still more preferably, from the viewpoint of improving the taste, 8% by mass or less. In the final product, the amount of (B) the indigestible dextrin is more preferably 1 to 10% by mass, still more preferably 4 to 8% by mass. An amount within this range is preferred from the viewpoint of production of the effect of the second embodiment.

The ratio between the amounts of (A) the milk protein hydrolysate and (B) the indigestible dextrin used in the second embodiment is not limited. From the viewpoint of better production of the effect of the second embodiment, they are contained in the final product at a mass ratio of preferably indigestible dextrin : milk protein hydrolysate = 10:0.001 to 1:1, more preferably indigestible dextrin : milk protein hydrolysate = 1:0.001 to 10:1.

The ratio between the amounts of (a4) the tripeptide MKP and (B) the indigestible dextrin used in the second embodiment is not limited. From the viewpoint of better production of the effect of the second embodiment, the amount of the indigestible dextrin with respect to 1 part by mass of the tripeptide MKP in the final product is, regarding the lower limit, preferably 5,000 parts by mass or more, more preferably 10,000 parts by mass or more, and, regarding the upper limit, preferably 100,000 parts by mass or less, more preferably 80,000 parts by mass or less. The amount of the indigestible dextrin is still more preferably 15,000 to 50,000 parts by mass with respect to 1 part by mass of the tripeptide MKP.

In the second embodiment, the amount of (C) sucralose in the final product is not limited. Regarding the lower limit, the amount is preferably 0.001% by mass or more, more preferably 0.004% by mass or more, still more preferably 0.007% by mass or more. Regarding the upper limit, the amount is preferably 0.5% by mass or less, more preferably 0.3% by mass or less, still more preferably 0.1% by mass or less. In the final product, the amount of (C) sucralose is more preferably 0.001 to 0.1% by mass, still more preferably 0.005 to 0.02% by mass. An amount within this range is preferred from the viewpoint of improving the taste of the fermented milk while masking the bitterness, starchy odor, and unpleasant odor without deteriorating the fermented milk-specific flavor.

In the second embodiment, the amount of (D) the monosaccharide in the final product is not limited. Regarding the lower limit, the amount is preferably 0.0005% by mass or more, more preferably 0.005% by mass or more, still more preferably 0.015% by mass or more. Regarding the upper limit, the amount is preferably 0.5% by mass or less, more preferably 0.25% by mass or less, still more preferably 0.1% by mass or less.

The amount of (D) the monosaccharide in the final product is more preferably 0.0005 to 0.5% by mass, still more preferably 0.005 to 0.25% by mass, still more preferably 0.015 to 0.1% by mass. A content within this range is preferred from the viewpoint of improving the fermented-milk flavor and the milk taste.

In the second embodiment, the amount of (D) the rare sugar-containing syrup in the final product is not limited. Regarding the lower limit, the amount is preferably 0.001% by mass or more, more preferably 0.01% by mass or more, still more preferably 0.03% by mass or more. Regarding the upper limit, the amount is preferably 1% by mass or less, more preferably 0.5% by mass or less, still more preferably 0.2% by mass or less. The amount of (D) the monosaccharide in the final product is more preferably 0.001 to 1% by mass, still more preferably 0.01 to 0.5% by mass, still more preferably 0.03 to 0.2% by mass. An amount within this range is preferred from the viewpoint of improving the taste of the fermented milk while masking the bitterness, starchy odor, and unpleasant odor without deteriorating the fermented milk-specific flavor.

In the second embodiment, (E) the lactulose is preferably included in the final product. The amount of (E) lactulose in the final product is not limited. Regarding the lower limit, the amount is preferably 0.1% by mass or more, more preferably 0.5% by mass or more, still more preferably 1% by mass or more. Regarding the upper limit, the amount is preferably 8% by mass or less, more preferably 6% by mass or less, still more preferably 4% by mass or less.

In the final product, the amount of (E) lactulose is more preferably 0.5 to 6% by mass, still more preferably 1 to 4% by mass. An amount within this range is preferred from the viewpoint of improving the fermented-milk flavor and the milk taste.

<2-3. Production Steps for Fermented Milk of Second Embodiment>

The production method of the second embodiment is preferably a method of producing a fermented milk, including the step of allowing fermentation of a liquid milk preparation containing (A) and/or (B), and also containing (C) and (D). (A) Milk protein hydrolysate; (B) indigestible dextrin; (C) sucralose; (D) one or more monosaccharides such as fructose, psicose, and allose.

In the production method of the second embodiment, the liquid milk preparation preferably further contains (E) lactulose.

The method of producing a fermented milk of the second embodiment is a method of producing a fermented milk containing (A) a milk protein hydrolysate and/or (B) an indigestible dextrin, the method preferably including a raw material preparation step (I) and a fermentation step (II). In the second embodiment, when necessary, heat treatment may be carried out in the raw material preparation step (I). In the second embodiment, when necessary, the fermentation step (II) may be followed by an addition step of adding one or more of the (A) to (E).

Each of the components (A) to (E) used in the production method of the second embodiment may be added, as a fermented-milk raw material, at least before the fermentation step or after the fermentation step, to the product in each production step as appropriate.

In the method of producing a fermented milk of the second embodiment, the (C) and (D) are preferably used as masking agents before the fermentation, from the viewpoint of reducing assimilation of the masking components. In the method of producing a fermented milk of the second embodiment, the (C) and (D); or the (C) and (D), and also (E); are preferably used after the fermentation, from the viewpoint of improving the taste of the fermented milk.

The fermentation raw material containing the (A) and/or (B), and also containing the (C) and (D); or the fermentation raw material containing the (A) to (D), and also containing the (E); used in the production method of the second embodiment is preferably prepared as a fermentation raw material solution, and then prepared into the liquid milk preparation, from the viewpoint of increasing the production efficiency in sterilization and the like.

The production method of the second embodiment may include use of one or more of (A) to (D), together or separately, before the fermentation and after the fermentation.

Further, the production method of the second embodiment may include use of the (E), before the fermentation and/or after the fermentation.

Each step of the production method of the second embodiment is described below in more detail.

In the second embodiment, the fermentation raw material is an unfermented raw material for use in the fermentation of the milk. The raw material, to be used for the production of the fermented milk, is obtained by heat-treating a milk-derived raw material, and then adding a bacterium/bacteria belonging to the genus *Bifidobacterium* and/or a lactic acid bacterial starter to the treated product. The fermentation raw material (hereinafter also referred to as "unfermented raw material") is not limited. The fermentation raw material may be one normally used for production of a fermented milk, or may be a commercially available product.

The milk-derived raw material is not limited as long as it is a milk or a milk product normally used for production of a fermented milk. Examples of the milk-derived raw material include cow's milk, buffalo milk, sheep milk, goat milk, horse milk, skim milk, concentrated skim milk, non-fat dry milk, concentrated milk, whole milk powder, cream, butter, buttermilk, condensed milk, and milk protein. One of these may be used, or two or more of these may be used as a mixture. The milk-derived raw material may also contain, when necessary, components normally used in production of a fermented milk, such as water, sweeteners, stabilizers, fats, additives, flavoring agents, fruits, and fruit sauces. As the water, a water normally used for production of foods is used.

Examples of the sweeteners include, but are not limited to, sugars such as glucose, table sugar, starch syrup, powdered starch syrup, and oligosaccharides; and high-intensity sweeteners such as acesulfame potassium, thaumatin, aspartame, alitame, and neotame, and stevioside, which is contained in stevia extracts.

Examples of the stabilizers include, but are not limited to, agar and gelatin.

Examples of the flavoring agents include, but are not limited to, natural perfumes and various flavors.

In the production of the fermented milk of the second embodiment, various components commonly used as fermentation raw materials (unfermented raw materials) or fermented-milk raw materials may be selected in addition to the (A) to (E) depending on the type of the fermented milk, and the amount of each of these may be adjusted.

According to the "Ministerial Ordinance on Milk and Milk Products Concerning Compositional Standards, etc." (hereinafter referred to as "Ministerial Ordinance on Milk, etc."), the fermented milks are defined as "the products which are obtained by fermenting milk, or milk, etc. containing an equal or greater amount of milk solids-not-fat with lactic acid bacteria or yeast and then forming a paste or liquid, or the frozen product". According to the composition standards, they satisfy the following: "milk solids-not-fat content, minimum 8%; lactic acid bacteria count or yeast count (per 1 mL), minimum 10,000,000; coliforms, negative". In the present description, the lactic acid bacteria count or bifidobacteria count is expressed with CFU (colony forming unit).

According to the Ministerial Ordinance on Milk, etc., the milk product lactic acid bacteria beverages are defined as "drinks which are obtained by fermenting milk, etc. with lactic acid bacteria or yeast and then processing it or using it as the principal ingredient (excluding fermented milk)". According to the composition standards, they satisfy the following: "milk solids-not-fat content, minimum 3%; lactic acid bacteria count or yeast count (per 1 mL), minimum 10,000,000; coliforms, negative".

According to the Ministerial Ordinance on Milk, etc., the lactic acid bacteria beverages which are foods using milk, etc. as principal ingredients are defined as "drinks which are obtained by fermenting milk, etc. with lactic acid bacteria or yeast and then processing it or using it as the principal ingredient (excluding fermented milk)". According to the composition standards, they satisfy the following: "milk solids-not-fat content, less than 3%; lactic acid bacteria count or yeast count (per 1 mL), minimum 10,000,000; coliforms, negative".

In the present description, "fermented milk" may be in any of a solid form, paste form, and liquid form. The fermented milk includes, in addition to the fermented milks defined by the Ministerial Ordinance on Milk, etc., any of the milk product lactic acid bacteria beverages, and the lactic acid bacteria beverages which are foods using milk, etc. as principal ingredients, defined by the Ministerial Ordinance on Milk, etc.

The types of the fermented milks can be roughly divided into (i) solid-form yogurt (stationary yogurt, post-fermented yogurt), prepared by filling the raw materials into a container and allowing fermentation, and (ii) liquid-form or paste-form yogurt (stirred yogurt, pre-fermented yogurt), prepared by placing the raw materials in a tank, allowing fermentation, crushing the resulting curd, and then easily filling the resulting product.

Examples of the stationary yogurt include plain yogurt and hard yogurt. Examples of the stirred yogurt include soft yogurt, drink yogurt, and frozen yogurt.

<2-3(1) Raw Material Preparation Step (1)>

The raw material preparation step (I) is a step of subjecting a fermentation raw material (unfermented raw material) solution to homogenization treatment, to prepare a liquid milk preparation.

Preferably, from the viewpoint of the work efficiency, the unfermented raw material solution contains (A) a milk protein hydrolysate and/or (B) an indigestible dextrin, and also contains (C) sucralose and (D) one or more monosaccharides such as fructose, psicose, and allose. The unfermented raw material solution preferably further contains (E) lactulose. Since the (A) to (E) are fermented-milk raw materials, these may be used as raw materials of the liquid milk preparation (unfermented raw materials) as described above. They may be used in any step in the production process of the fermented milk. They may be used after the fermentation rather than before the fermentation.

The fermentation raw material solution (unfermented raw material solution) in the second embodiment is a solution obtained by dissolving fermentation raw materials (unfermented raw materials) and the like in a solvent.

The method of preparing the solution by dissolving the fermentation raw materials (unfermented raw materials) in the solvent is not limited, and the preparation may be carried out by a method normally used.

The solvent is not limited, and one of, or a combination of two or more of, the known solvents may be used. Specific examples of the solvent include water and warm water.

The temperature of the solvent during the dissolution is not limited as long as the effect of the second embodiment is not deteriorated, and may be arbitrarily set.

Further, when necessary, a power blender, mixer, high-speed stirrer, or the like may be used as a dissolver.

In the second embodiment, the fermentation raw material solution (unfermented raw material solution) may contain a sweetener, stabilizer, flavoring agent, or the like as long as the effect of the second embodiment is not deteriorated.

The "liquid milk preparation" in the second embodiment is not limited as long as it is prepared by homogenization treatment of the fermentation raw material solution (unfermented raw material solution). Its concept also includes the product after the later-mentioned heat treatment.

The method of the homogenization treatment in the raw material preparation step (1) is not limited, and the treatment may be carried out by a method normally used. Specific examples of the method of the homogenization treatment include a method in which a pressure of about 10 to 20 MPa is applied using a homogenizer or the like to a solution heated at 65 to 90°C.

In the second embodiment, the timing of performing the homogenization treatment is not limited. The homogenization treatment may be carried out before the later-mentioned heat treatment, or may be carried out after the heat treatment, but before the fermentation step (II). From a sanitary point of view, the homogenization treatment is preferably carried out before the heat treatment.

In the second embodiment, in the raw material preparation step (I), the homogenization treatment of the fermentation raw material solution (unfermented raw material solution) may be followed by heat treatment at 70 to 100°C for 5 to 15 minutes. By this, a sterilization effect by the heat treatment can be obtained.

It is generally known that the conditions for the heat treatment in production of a fermented milk are normally as follows: (i) at a temperature of 85°C for 30 minutes; (ii) at a temperature of 90 to 95°C for 10 minutes to 5 minutes; (iii) at a temperature of 120°C for 5 to 3 seconds (see, for example, p. 282 in New Food Processing Lecture: Milk and Its Processing (saishin shokuhinkakokoza: nyu to sono kako), First Edition, published by Kenpakusha Co., Ltd. (1987)).

The method of the heat treatment in the raw material preparation step (I) is not limited, and the heat treatment may be carried out by a method normally used. More specifically, for example, the heat treatment of the fermentation raw material solution (unfermented raw material solution) may be carried out using, for example, a heat treatment apparatus such as a plate-type sterilizer, tubular-type sterilizer, direct heating-type sterilizer, or jacketed tank.

In the second embodiment, the heat treatment may be followed by cooling of the liquid milk preparation to a predetermined temperature. More specifically, for example, the liquid milk preparation is cooled to a temperature near the fermentation temperature in the later-mentioned fermentation step (II).

<2-3(2) Fermentation Step (II)>

The fermentation step (II) is a step of adding a milk fermentation bacterium/bacteria (more specifically, a lactic acid bacterium/bacteria, a bacterium/bacteria belonging to the genus *Bifidobacterium,* and/or the like) to the liquid milk preparation, to allow fermentation.

In the second embodiment, the milk fermentation bacterium/bacteria added to the liquid milk preparation, and the fermentation conditions (fermentation temperature, time, and the like) are not limited, and those normally used may be employed.

In general, "lactic acid bacteria" is a general term for the bacteria belonging to the genus *Lactococcus,* the bacteria belonging to the genus *Lactobacillus,* and the bacteria belonging to the genus *Streptococcus,* which bacteria produce lactic acid by metabolism. The bacteria belonging to the genus *Bifidobacterium* are also called "bifidobacteria", which is a general term for the bacteria that produce lactic acid and acetic acid by metabolism.

These lactic acid bacteria are also called the starter (seed bacteria). A commercially available starter may be used.

Examples of the bacteria belonging to the genus *Lactococcus* include the bacterial strains of *Lactococcus lactis* (L. *lactis), Lactococcus lactis* subsp. *lactis* (L. *lactis* subsp. *lactis*), *Lactococcus lactis* subsp. *lactis* biovar. diacetylactis (*L. lactis* subsp. *lactis* biovar. diacetylactis), *Lactococcus lactis* subsp. *cremoris* (*L. lactis* subsp. *cremoris*), and the like;

examples of the bacteria belonging to the genus *Lactobacillus* include the bacterial strains of *Lactobacillus delbrueckii* subsp. *lactis* (*L. delbrueckii* subsp. *lactis*), *Lactobacillus delbrueckii* subsp. *bulgaricus* (*L. delbrueckii* subsp. *bulgaricus*)*, Lactobacillus bulgaricus* (*L. bulgaricus*)*, Lactobacillus reuteri* (*L. reuteri*), *Lactobacillus helveticus* (*L. helveticus*)*,* and the like; and examples of the bacteria belonging to the genus *Streptococcus* include the bacterial strains of *Streptococcus salivarius* subsp. *thermophilus* (*S. salivarius* subsp. *thermophilus*), *Streptococcus thermophilus* (*S. thermophilus*), and the like. One of, or a combination of two or more of, these bacteria may be used.

As the starter, from the viewpoint of the production efficiency of the fermented milk and the effect of the fermented milk, a bacterium/bacteria belonging to the genus *Lactobacillus* and/or a bacterium/bacteria belonging to the genus *Streptococcus* is/are preferred. One or more of these examples are more preferred.

Further, the starter in the second embodiment may be one or more of *Lactobacillus delbrueckii* subsp. *bulgaricus, Lactobacillus bulgaricus, Streptococcus salivarius* subsp. *thermophilus,* and *Streptococcus thermophilus.*

The starter is more preferably the combination of *Lactobacillus delbrueckii* subsp. *bulgaricus* and *Streptococcus thermophilus.* This allows better production of the effect and taste of the fermented milk with the combination of (i) the (A) to (D); or (ii) the (A) to (D) and the (E). In cases where (A) the milk protein hydrolysate is used as a fermentation raw material, a starter that does not assimilate the tripeptide MKP is preferably selected so that the effect of the MKP can be expected.

Examples of the bacteria belonging to the genus *Bifidobacterium* include the bacterial strains of *Bifidobacterium longum* (*B. longum*), *Bifidobacterium breve* (*B. breve*), *Bifidobacterium bifidum* (*B. bifidum*), *Bifidobacterium infantis* (*B. infantis*), *Bifidobacterium animalis* (*B. animalis*), and the like. One of, or a combination of two or more of, these bacteria may be used.

The amount of these lactic acid bacteria added to the liquid milk preparation may be appropriately adjusted within an ordinary range. For example, they are preferably added in an amount with which the bacteria concentration in the liquid milk preparation becomes at least about $1\times10^5$ CFU/g, preferably at least about $1\times10^7$ CFU/g.

The method of adding the bacterium/bacteria to the liquid milk preparation is not limited, and examples of the method include a method in which the bacterium/bacteria is/are added in the state of a bacteria powder, and a method in which the bacterium/bacteria is/are added in the state of a culture (cultured product).

The fermentation temperature is not limited, and may be within a range in which bacteria such as lactic acid bacteria efficiently grow. The temperature is usually about 30 to 50°C, preferably about 35 to 43°C. The fermentation time is not limited, and is about 3 to 10 hours, preferably about 3 to 7 hours.

Regarding the pH during the fermentation, the fermentation may be carried out usually until the pH of the liquid milk preparation becomes 5.0 or less, preferably until the pH of the liquid milk preparation becomes 4.8 or less, more preferably until the pH of the liquid milk preparation becomes about 4.2 to 4.8.

In the second embodiment, after the fermentation step (II), the fermented liquid milk preparation may be cooled to a predetermined temperature. More specifically, for example, the liquid milk preparation is cooled to 10°C or less.

In cases where a stationary yogurt is produced as the fermented milk, the liquid milk preparation containing the fermentative bacterium/bacteria may be added to a container, and may then be fermented. In cases where a stirred yogurt is produced as the fermented milk, the fermentation step (II) may be followed by crushing the resulting curd, and then adding the crushed curd to a container. Examples of the container include plastic containers and paper containers.

(3) Other Steps

In the second embodiment, other steps may be carried out as long as the effect of the second embodiment is not deteriorated.

Examples of the other steps include an addition step of adding any of, or a combination of, the (A) to (E) after the fermentation step. Examples of the other steps also include an addition step of adding a stabilizer, additive, flavoring agent, fruit, fruit sauce, or the like.

<2-4. Use of Fermented Milk According to Second Embodiment for Improvement of Taste>

The second embodiment can also provide a method of improving the taste of a fermented milk containing (A) the milk protein hydrolysate and/or (B) the indigestible dextrin, the method including: using (C) the sucralose and (D) the one or more monosaccharides such as fructose, psicose, and allose. As the (D), a rare sugar(s) may be used. Preferably, (E) the lactulose is also used.

The taste improver for a fermented milk in the second embodiment is a taste improver for a fermented milk containing (A) the milk protein hydrolysate and/or (B) the indigestible dextrin, the taste improver including: (C) the sucralose and (D) the one or more monosaccharides selected from the group consisting of fructose, psicose, and allose. The taste improver preferably further contains (E) the lactulose.

The (C) and (D), or the (C) to (E) in the second embodiment are preferably used for improvement of the taste of the fermented milk containing the (A) and/or the (B), in any of the steps of the <2. Method of Producing Fermented Milk Containing Milk Protein Hydrolysate and/or Indigestible Dextrin>. (A) The milk protein hydrolysate and/or (B) the indigestible dextrin may be used in any of the steps of the <2. Method of Producing Fermented Milk Containing Milk Protein Hydrolysate and/or Indigestible Dextrin>, to include it/them in the final product. It/They may be used before the fermentation and/or after the fermentation.

The timing of use or addition of the (C) and (D) (preferably at least the three components (C), (D), and (E)) in the second embodiment is not limited as long as the effect of the second embodiment is not deteriorated. Each of these may be used or added before the fermentation and/or after the fermentation.

The (C) and (D) (preferably at least the three components (C), (D), and (E)) in the second embodiment are preferably added, together or separately, as fermented-milk raw materials before the fermentation, from the viewpoint of the work efficiency. More preferably, the (C) and (D) (preferably at least the three components (C), (D), and (E)) in the second embodiment are used as liquid-milk-preparation raw materials or a liquid milk preparation.

The (C) and (D) (preferably at least the three components (C), (D), and (E)) in the second embodiment are preferably added, together or separately, as fermented-milk raw materials after the fermentation, from the viewpoint of improvement of the taste.

The (A) in the second embodiment is preferably a casein hydrolysate.

In the second embodiment, the method of using each of the (A) to (E), and the amount of each of the (A) to (E) used, are as described in the <2. Method of Producing Fermented Milk Containing Milk Protein Hydrolysate and/or Indigestible Dextrin>.

The (C) and (D) (preferably at least the three components (C), (D), and (E)) in the second embodiment may be used for improvement of the taste of a fermented milk using the (A) and/or (B). Thus, the (C) and (D) (preferably at least the three components (C), (D), and (E)) in the second embodiment may be included as effective components in the fermented milk using the (A) and/or (B) for the purpose of improving the taste of the fermented milk, or may be materials or formulations to be used for the fermented milk or the like.

The (C) and (D) (preferably at least the three components (C), (D), and (E)) in the second embodiment may be used as they are, or may be used as a mixture with a sitologically acceptable ordinary carrier or diluent, for the (A) and/or (B).

The (C) and (D) (preferably at least the three components (C), (D), and (E)) in the second embodiment may be used for production of a composition, formulation, or the like for improvement of the taste of a fermented milk using the (A) and/or (B).

The second embodiment can provide a fermented milk in which, even in cases where a milk protein hydrolysate and/or an indigestible dextrin is/are used as a fermented-milk raw material(s), the fermented milk-specific flavor can be maintained as much as possible while favorably masking the bitterness, starchy odor, and unpleasant odor derived from the milk protein hydrolysate and/or the indigestible dextrin. This allows improvement of the taste of the fermented milk that has been deteriorated due to the milk protein hydrolysate and/or the indigestible dextrin.

<2-5. Fermented Milk According to Second Embodiment>

By inclusion of the combination of (C) the sucralose and (D) the one or more monosaccharides such as fructose, psicose, and allose, the fermented milk of the second embodiment can have a pleasant fermented-milk taste without deterioration of the fermented milk-specific flavor, while favorably masking the bitterness, starchy odor, and unpleasant odor derived from (A) the milk protein hydrolysate and/or (B) the indigestible dextrin.

Moreover, by the additional inclusion of (E) the lactulose, the fermented milk of the second embodiment can have a fermented-milk taste with a better fermented-milk flavor and milk taste.

The fermented milk of the second embodiment may be, but does not necessarily need to be, produced as in the <2. Method of Producing Fermented Milk Containing Milk Protein Hydrolysate and/or Indigestible Dextrin>.

Since the fermented milk of the second embodiment is characterized in that it contains (A) the milk protein hydrolysate and/or (B) the indigestible dextrin, effects of the milk protein hydrolysate and/or the indigestible dextrin can be expected. Further, with the fermented milk of the second embodiment, effects (such as a probiotic effect, an intestinal function-controlling action, and/or an immunostimulatory action) of the milk fermentative bacterium/bacteria (such as lactic acid bacteria and/or bifidobacteria) in the fermented milk can be expected.

Thus, since the fermented milk of the second embodiment has a pleasant fermented-milk taste, it can be practically used as a food, beverage, or the like that can be ingested on a daily basis for the purpose of maintenance and promotion of health.

Since the fermented milk of the second embodiment uses (C) the sucralose and (D) the one or more monosaccharides such as fructose, psicose, and allose (preferably at least the three components (C), (D), and (E)) for improvement of the taste of the fermented milk, the amount of sugars commonly used as fermented-milk raw materials (preferably carbohydrates utilized as energy sources, such as glucose) used can be reduced. With the fermented milk of the second embodiment, carbohydrates can be preferably reduced by about 30%, more preferably reduced by 40% compared to common fermented milks.

Thus, the fermented milk of the second embodiment may be practically used as a composition (for a pharmaceutical, food or beverage, feed, or the like) for the purpose of prevention, amelioration, and/or treatment of a lifestyle-related disease (such as obesity, diabetes, hypertension, hyperlipidemia, complication, or a disease or symptom caused thereby), or for the purpose of maintenance or promotion of health.

More specifically, the fermented milk of the second embodiment may be practically used as, for example, a health food; functional food; food with function claims; patient food; enteral food; special purpose food; food with health claims; food for specified health uses whose label indicates, for example, uses for individuals who are concerned about the blood glucose level, for individuals who are concerned about obesity, for individuals who are concerned about cholesterol or neutral fat, for individuals who are concerned about salt intake, or for individuals who are interested in prevention of lifestyle-related diseases; food with functional claims; or food with nutrient function claims; intended for uses such as suppression of elevation of the blood glucose level, lowering of the blood pressure, reduction of the blood neutral fat, or suppression of elevation of the blood cholesterol.

The term used for the above labeling is not limited to the term "lifestyle-related diseases". Other terms, of course, are also included within the scope of effect of the second embodiment as long as the terms indicate effects for prevention, amelioration, and/or treatment of diseases and symptoms related to lifestyle-related diseases. Examples of the term include those for labels based on various uses that allow the consumers to recognize effects such as prevention, amelioration, and/or the like of lifestyle-related diseases.

In the second embodiment, the contents of the (A) to (E) in the fermented milk are as described in the <2-2-2. Amounts of Components (A) to (E) in Fermented-Milk Final Product> in the <2. Method of Producing Fermented Milk Containing Milk Protein Hydrolysate and/or Indigestible Dextrin>.

In the second embodiment, the amount of (A) the milk protein hydrolysate in the fermented milk is not limited. From the viewpoint of production of the effect of the second embodiment, it is preferably 0.1 to 1.0% by mass.

In the second embodiment, the content of the tripeptide MKP in the fermented milk is not limited. From the viewpoint of production of the effect of the second embodiment, it is preferably 0.00009 to 0.0008% by mass.

In the second embodiment, the content of (B) the indigestible dextrin in the fermented milk is not limited. From the viewpoint of production of the effect of the second embodiment, it is preferably 1 to 10% by mass.

In the second embodiment, in cases where the fermented milk contains (A) the milk protein hydrolysate and (B) the indigestible dextrin, the mass ratio between (A) the milk protein hydrolysate and (B) the indigestible dextrin is not limited. From the viewpoint of better production of the effect of the second embodiment, they are more preferably present at a mass ratio of indigestible dextrin : milk protein hydrolysate = 1:0.001 to 10:1.

In the second embodiment, the mass ratio between the amounts of (a4) the tripeptide MKP and (B) the indigestible dextrin in the fermented milk is not limited. From the viewpoint of better production of the effect of the second embodiment, the amount of the indigestible dextrin is preferably 15,000 to 50,000 parts by mass with respect to 1 part by mass of the tripeptide MKP.

In the second embodiment, the content of (C) sucralose in the fermented milk is not limited. From the viewpoint of improving the taste of the fermented milk while masking the bitterness, starchy odor, and unpleasant odor without deteriorating the fermented milk-specific flavor, the content is preferably 0.001 to 0.1% by mass.

In the second embodiment, the content of (D) the one or more monosaccharides such as fructose, psicose, and allose in the fermented milk is not limited. From the viewpoint of improving the taste of the fermented milk while masking the bitterness, starchy odor, and unpleasant odor without deteriorating the fermented milk-specific flavor, the content is preferably 0.015 to 0.1% by mass.

In the second embodiment, the fermented milk preferably includes (E) lactulose. The amount of (E) lactulose in the fermented milk is not limited, and is preferably 1 to 4% by mass.

In the second embodiment, the amount of the lactic acid bacterium/bacteria in the fermented milk is preferably $1\times10^5$ to $1\times10^{12}$ CFU/mL, more preferably $1\times10^6$ to $1\times10^{11}$ CFU/mL, still more preferably $1\times10^7$ to $1\times10^{10}$ CFU/mL.

In the second embodiment, the amount of the bacterium/bacteria belonging to the genus *Bifidobacterium* in the fermented milk is preferably $0.5\times10^8$ to $10\times10^8$ CFU/mL, more preferably $1\times10^8$ to $10\times10^8$ CFU/mL.

The present technique may also employ the following:

[1] A composition for lowering blood pressure and/or reducing neutral fat; or a composition for prevention, amelioration, and/or treatment of hypertension and/or dyslipidemia (hyperlipidemia); the composition comprising a casein hydrolysate and an indigestible dextrin.

[2] A method of lowering of the blood pressure and/or reduction of neutral fat; or a method of prevention, amelioration, and/or treatment of hypertension and/or dyslipidemia (hyperlipidemia); the method comprising administration or ingestion of a casein hydrolysate and an indigestible dextrin.

[3] A casein hydrolysate and an indigestible dextrin, to be used for lowering of the blood pressure and/or reduction of neutral fat; or to be used for prevention, amelioration, or treatment of hypertension and/or dyslipidemia (hyperlipidemia); or use thereof.

[4] Use of a casein hydrolysate and an indigestible dextrin, for production of a composition for lowering blood pressure and/or reducing neutral fat; or for production of a composition for prevention, amelioration, or treatment of hypertension and/or dyslipidemia (hyperlipidemia).

[5] Preferably, the casein hydrolysate in any one of [1] to [4] comprises a peptide composed of Met-Lys-Pro.

[6] Preferably, the indigestible dextrin and the casein hydrolysate are contained at a mass ratio of 10:0.001 to 1:1 in any one of [1] to [5].

[7] Preferably, the casein hydrolysate in any one of [1] to [6] comprises the peptide of Met-Lys-Pro. More preferably, the amount of the indigestible dextrin is 5,000 to 100,000 parts by mass with respect to 1 part by mass of the peptide.

[8] Preferably, in any one of [1] to [7], the composition is for a pharmaceutical or for a food or beverage.

[9] Preferably, in any one of [1] to [8], the composition is for a fermented food or beverage; more preferably, the composition is a fermented food or beverage.

[10] Preferably, in any one of [1] to [9], the composition is in the form of a unit package.

[11] Preferably, in any one of [1] to [10], the composition is in the form of a powder or granule, a tablet formed by compression, or a tablet characterized in that the composition is filled in a capsule, or these are used.

[12] Preferably, in any one of [1] to [11], the casein hydrolysate is present at 0.001 to 1% by mass with respect to the mass of the final composition.

[13] Preferably, in any one of [1] to [12], the indigestible dextrin is present at 1 to 10% by mass with respect to the mass of the final composition.

[14] Preferably, in any one of [9] to [13], the fermented food or beverage comprises sucralose, and one or more monosaccharides and/or rare sugars selected from the group consisting of fructose, psicose, and allose.

[15] In [14], the fermented food or beverage further comprises lactulose.

[16] A method of lowering of the blood pressure and/or reduction of neutral fat; or a method of prevention, amelioration, and/or treatment of hypertension and/or dyslipidemia (hyperlipidemia); using the composition in any one of [1] to [15]-[17]

A method of producing a fermented milk which is a method of producing a fermented food or beverage containing (A) a casein hydrolysate and (B) an indigestible dextrin, the method comprising: further including (C) sucralose, and (D) one or more monosaccharides and/or rare sugars selected from the group consisting of fructose, psicose, and allose.

[18] The method of producing a fermented milk according to [17], wherein the casein hydrolysate contains a peptide composed of Met-Lys-Pro.

[19] The method of producing a fermented milk according to [17] or [18], wherein the fermented milk further contains lactulose.

Examples

The present technique is described below in more detail by way of Examples and Test Examples. The Examples, Test Examples, and the like described below show representative examples of the present technique. Interpretation of the scope of the present technique is not narrowed by these.

Test Related to Blood Pressure-Lowering Action and/or Neutral Fat-Reducing Action in Present Technique

Production Example 1

To 100 mg of a commercially available casein (manufactured by New Zealand Dairy Board), 900 mg of water was added, and the casein was sufficiently dispersed, followed by adding sodium hydroxide thereto to adjust the pH of the resulting solution to 7.0. By allowing complete dissolution of the casein, an aqueous casein solution at a concentration of about 10% was prepared.

The aqueous casein solution was heat-sterilized at 85°C for 10 minutes, and then the temperature was adjusted to 50°C, followed by adding sodium hydroxide thereto to adjust the pH to 9.0. Thereafter, 2 mg of pancreatin (manufactured by Amano Enzyme Inc.) and 4 mg of protease A (manufactured by Amano Enzyme Inc.) were added thereto to start hydrolysis reaction. Eight hours later, the solution was heated at 80°C for 6 minutes to deactivate the enzymes, to stop the enzyme reaction. The solution was then cooled to 10°C.

The resulting hydrolysate was subjected to ultrafiltration using an ultrafiltration membrane having a molecular weight cutoff of 1,000 (manufactured by Nihon Pall Ltd.), and then concentrated and freeze-dried, to obtain 85 mg of a casein digest.

The above process was carried out for a plurality of lots. As a result, the casein hydrolysate showed a degradation rate of 20 to 30%, average molecular weight of 800 Da or less, free amino acid ratio of 10% or less, and a tripeptide MKP concentration within the range of 0.01 to 0.1% by mass. These were calculated according to the <Degradation Rate of Amino Acids>, <Method of Calculating Average Molecular Weight>, <Method of Calculating Free Amino Acid Ratio>, and <Measurement of Tripeptide MKP Content>. The average molecular weight of the casein hydrolysate can be adjusted to 360 to 390 Da.

Example 1

In the present Example, changes in serum triglyceride (TG) in rats upon loading with lipid were evaluated.

(1) Sample Preparation

Test Sample 1 + Test Sample 2 were prepared such that the casein hydrolysate (Test Sample 1) alone is contained at 1% by mass, and such that an indigestible dextrin (Fibersol-2, manufactured by Matsutani Chemical Industry Co., Ltd.) (Test Sample 2) alone is contained at 10% by mass, in water for injection (manufactured by Otsuka Pharmaceutical Factory, Inc.). As a control sample, the water for injection was used.

(2) Test Method

Male Slc:SD (SPF) rats of 6 weeks old were purchased from Japan SLC Inc. After the rats were preliminary kept for 1 week with Labo MR Stock (Nosan Corporation), eight mice were assigned to each of Group A (control) and Group B (administration of Test Sample 1 + Test Sample 2). From the evening on the day before the lipid loading test, the rats were fasted overnight (for about 16 hours), and then blood was collected from the tail vein (blood collection at Hour 0). The control sample or the test samples was/were administered by forced oral administration at 10 mL/kg, which was immediately followed by forced oral administration of corn oil at a ratio of 3 g/kg. Blood was collected at Hour 2, Hour 4, Hour 5, and Hour 6 after the administration. The blood obtained at each time point was centrifuged after the blood collection, and the serum was collected. The serum obtained was subjected to measurement of blood triglyceride using a Type-7070 Autoanalyzer (Hitachi, Ltd.)

(3) Test Results

The changes in the serum TG level are shown in Table 1. Each value in the table is expressed as the mean and the standard error. Concerning significant differences, homoscedasticity was tested by the Bartlett method, and, in homoscedastic cases, one-way analysis of variance was carried out. In significant cases, comparison of the means was carried out by the Dunnett's method.

Table 1

|  |  | Time (Hours) | | | | |
|---|---|---|---|---|---|---|
|  |  | 0 | 2 | 4 | 5 | 6 |
| A | Control | 38±3.2 | 172.9±22.7 | 250.9±44.9 | 229.4±47.2 | 117.9±18.2 |
| B | Test Sample 1 + Test Sample 2 | 36±3.1 | 135.6±13.4 | 190±36.8 | 130.8±23.0 (p<0.1) | 118.3±24.7 |

As shown in Table 1, suppression of an increase in blood TG was found in Group B compared to Group A.
From this result, it was found that combined use of a casein hydrolysate and an indigestible dextrin produces an effect which suppresses an increase in blood lipid.

Example 2

In the present Example, a blood pressure-lowering effect in hypertensive rats was evaluated.

(1) Sample Preparation

Test Sample 1 + Test Sample 2 were prepared such that the casein hydrolysate (Test Sample 1) alone is contained at 1% by mass, and such that an indigestible dextrin (Fibersol-2, manufactured by Matsutani Chemical Industry Co., Ltd.) (Test Sample 2) alone is contained at 10% by mass, in water for injection (manufactured by Otsuka Pharmaceutical Factory, Inc.). As a control sample, the water for injection was used.

(2) Test Method

Male SHR/Hos (SPF) rats of 10 weeks old were purchased from Japan SLC Inc. After the rats were preliminary kept for 1 week, six mice were assigned to each of Group A (control) and Group B (administration of Test Sample 1 + Test Sample 2). The animals after the grouping were subjected to measurement of the blood pressure before the beginning of the administration and at Hour 2, Hour 4, Hour 6, Hour 8, and Hour 24 after the administration. Regarding the blood pressure, the tail arterial-venous pressure was measured for the systolic blood pressure using a non-invasive automatic blood pressure measuring apparatus (BP-98A, Softron Co., Ltd.) by the tail cuff method under unanesthetized conditions. The measurement was carried out three times, and the mean value was regarded as the measured value for each individual. Before the measurement, the animals were placed in a warming box (set temperature, 38°C) for about 5 minutes in order to raise the body temperature of the animals.

(3) Test Results

The changes in the systolic blood pressure are shown in Table 2. For the table significance test, a two-group-comparison test was carried out. Uniformity of dispersion in two groups was tested by the F-test, and, in homoscedastic cases, the Student's t-test was carried out. Regarding the level of significance, significance was assumed at a significance level of less than 5% in all tests. Levels of significance were distinctly indicated for those of less than 5% ($p < 0.05$) and those of less than 1% ($p < 0.01$).

Table 2

| | | Systolic blood pressure value (mmHg) | | | | |
|---|---|---|---|---|---|---|
| | | | Time (Hours) | | | |
| | | Value before administration | 2 | 4 | 5 | 6 |
| A | Control | 198±2 | 188±2 | 181±3 | 178±2 | 177±2 |
| B | Test Sample 1 + Test Sample 2 | 196±4 | 185±3 | 174±4 | 167**±2 | 165**±3 |

**$p < 0.05$

As shown in Table 2, lowering of the blood pressure was found in Group B compared to Group A.
From this result, it was found that combined use of a casein hydrolysate and an indigestible dextrin produces a blood pressure effect.

Formulation Example 1: Fermented Milk for Lowering Blood Pressure and/or for Reducing Neutral Fat

Cow's milk or skim milk is blended with the casein hydrolysate of Production Example 1 and indigestible dextrin, and sucralose (manufactured by San-Ei Gen F.F.I., Inc.) and rare monosaccharide (trade name: Rare Sugar Sweet (manufactured by Matsutani Chemical Industry Co., Ltd.)), to prepare a liquid milk preparation containing (A) milk protein at 3.5% by mass or more, (B) milk fat at 3.5% by mass or less, (C) carbohydrate at 5.0% by mass or more, and (D) calcium at 0.15% by mass or more.
The liquid milk preparation is uniformly mixed with a lactic acid bacterium/bacteria and a bifidobacterium/bacteria, and the resulting mixture is fermented. By this, a composition for lowering blood pressure and/or reducing neutral fat (fermented milk) containing the casein hydrolysate and the indigestible dextrin is obtained. The fermented milk contains (E) the casein hydrolysate in Example 1 at 0.3 to 0.8% by mass, (F) indigestible dextrin at 4 to 8% by mass, (G) sucralose at 0.05 to 0.02% by mass, (H) D-psicose and D-allose at 0.05 to 0.1% by mass, and (I) lactulose at 1 to 4% by mass.
The fermented milk for lowering of the blood pressure and/or reduction of neutral fat of the present technique is continuously ingested on a daily basis such that the daily intake of casein hydrolysate is 0.0001 to 1 g/kg body weight/day, and such that the daily intake of indigestible dextrin is 0.001 to 10 g/kg body weight/day. By this, a blood pressure-lowering and/or neutral fat-reducing effect can be expected. Further, this enables providing of a fermented milk which can be expected to have an effect allowing easier ingestion of the fermented milk since the fermented milk-specific flavor can be maintained as much as possible while masking the bitterness, starchy odor, and unpleasant odor derived from the casein hydrolysate and/or the indigestible dextrin.

Formulation Example 2: Powder for Lowering Blood Pressure and/or for Reducing Neutral Fat

The components (powders) shown in Table 3 are mixed to obtain a powder for lowering blood pressure and/or for reducing neutral fat. The resulting powder may be used as a supplement, or may be mixed with water to provide a beverage. Further, the powder may be filled into a capsular container or encapsulated with a film, to provide a capsule for lowering blood pressure and/or for reducing neutral fat. Further, the powder may be compression-molded to provide a tablet confectionery for lowering the blood pressure and/or for reducing neutral fat. With this powder, a blood pressure-lowering and/or neutral fat-reducing effect can be expected. As raw materials of the powder, the casein hydrolysate of Production Example 1 and indigestible dextrin may be used.

Table 3

| Raw material (powder) | Blending ratio [% by mass] | Blended amount [mg/package] |
| --- | --- | --- |
| Indigestible dextrin | 85.000 | 5,100 |
| Casein digest | 8.500 | 510 |
| Flavoring formulation | 4.500 | 270 |
| Tricalcium phosphate | 1.000 | 60 |
| Sweetener formulation | 1.000 | 60 |
| Total | 100.000 | 6,000 |

Test Related to Method of Producing Fermented Milk of Second Embodiment Production Example 1 of Second Embodiment: Casein Hydrolysate

As (A) a milk protein hydrolysate, a casein hydrolysate was used.

To 100 mg of a commercially available casein (derived from cow's milk, manufactured by New Zealand Dairy Board), 900 mg of water was added, and the casein was sufficiently dispersed, followed by adding sodium hydroxide thereto to adjust the pH of the resulting solution to 7.0. By allowing complete dissolution of the casein, an aqueous casein solution at a concentration of about 10% was prepared.

The aqueous casein solution was heat-sterilized at 85°C for 10 minutes, and then the temperature was adjusted to 50°C, followed by adding sodium hydroxide thereto to adjust the pH to 9.0. Thereafter, 2 mg of pancreatin (manufactured by Amano Enzyme Inc.) and 4 mg of protease A (manufactured by Amano Enzyme Inc.) were added thereto to start hydrolysis reaction. Eight hours later, the solution was heated at 80°C for 6 minutes to deactivate the enzymes, to stop the enzyme reaction. The solution was then cooled to 10°C.

The resulting hydrolysate was subjected to ultrafiltration using an ultrafiltration membrane having a molecular weight cutoff of 1,000 (manufactured by Nihon Pall Ltd.), and then concentrated and freeze-dried, to obtain 85 mg of a casein digest.

(A) The casein hydrolysate was subjected to the above process for a plurality of lots. As a result, the casein hydrolysate showed a degradation rate of 20 to 30%, average molecular weight of 800 Da or less, free amino acid ratio of 10% or less, and a tripeptide MKP concentration within the range of 0.01 to 0.1% by mass. These were calculated according to the <Degradation Rate of Amino Acids>, <Method of Calculating Average Molecular Weight>, <Method of Calculating Free Amino Acid Ratio>, and <Measurement of Tripeptide MKP Content>. The average molecular weight of the casein hydrolysate can be adjusted to 360 to 390 Da.

As fermented-milk raw materials, the following components were used.

As a concentrated skim milk, Concentrated Skim Milk (manufactured by Morinaga Milk Industry Co., Ltd.) was used.

As a cream, Cream (manufactured by Morinaga Milk Industry Co., Ltd.) was used.

As (A) a milk protein hydrolysate, the casein hydrolysate of Production Example 1 was used.

As (B) an indigestible dextrin, Fibersol-2 (manufactured by Matsutani Chemical Industry Co., Ltd.) was used.

As (C) sucralose, Sucralose (manufactured by San-Ei Gen F.F.I., Inc.) was used.

As (D) a rare sugar-containing syrup, Rare Sugar Sweet (manufactured by Matsutani Chemical Industry Co., Ltd.) was used. The rare sugar-containing syrup contained 31% by mass D-fructose, 44% by mass D-glucose, 6% by mass D-psicose, and 4% by mass D-allose.

As (E) a lactulose-containing syrup, Milk Oligosaccharide MLS[(R)]-50 (manufactured by Morinaga Milk Industry Co., Ltd.) was used. The lactulose-containing syrup (milk oligosaccharide) contained 50% by mass lactulose.

As a flavoring agent, Yogurt Flavor (manufactured by San-Ei Gen F.F.I., Inc.) was used.

As a lactic acid bacterial starter, YO-MIX (manufactured by Danisco) was used. YO-MIX (manufactured by Danisco) contains *Streptococcus thermophilus* (*S. thermophilus*) and *Lactobacillus delbrueckii* subsp. *bulgaricus* (*L. delbrueckii* subsp. *bulgaricus*), and sucrose and maltodextrin.

Test Examples 1 to 20: Production of Fermented Milk: Drink Yogurt

The raw materials used for the production of the fermented milk: drink yogurt (stirred yogurt) in each of Test Examples 1 to 20 are shown in Table 4 to Table 7. The raw material components were mixed together to achieve 100% by mass in dissolved water.

The liquid milk preparation used in Test Example 20 contains (A) 0.50% by mass casein hydrolysate, (B) 5.00% by mass indigestible dextrin, (C) 0.01% by mass sucralose, (D) 0.05% by mass rare sugar-containing syrup (0.022% by mass D-glucose, 0.0155% by mass D-fructose, 0.003% by mass D-psicose, and about 0.002% by mass D-allose in the fermented-milk raw material), and (E) 2.00% by mass lactulose-containing syrup (1.00% by mass lactulose in the fermented-milk raw material).

| Table 4: Drink yogurt (pre-fermented) | | % by mass |
|---|---|---|
| | Test Example 1 (C1) | Test Example 2 (C2) |
| Raw material name | Blending % | Blending % |
| Concentrated skim milk | 24.00 | 24.00 |
| Cream | 0.50 | 0.50 |
| (A) Casein hydrolysate | - | 0.50 |
| (B) Indigestible dextrin | - | 5.50 |
| (C) Sucralose | - | 0.01 |
| (D) Rare sugar-containing syrup | - | 0.05 |
| (E) Lactulose-containing syrup | - | 2.00 |
| Starter | 0.01 | 0.01 |
| Water | 65.49 | 57.43 |
| Subtotal | 90 | 90 |
| | | |
| Pectin | 0.10 | 0.10 |
| Water | 9.90 | 9.90 |
| Subtotal | 10 | 10 |
| Total | 100.00 | 100.00 |

| Table 5: Drink yogurt (pre-fermented) | | | | | | % by mass |
|---|---|---|---|---|---|---|
| | Test Example 3 (M1) | Test Example 4 (M2) | Test Example 5 (M3) | Test Example 6 (M4) | Test Example 7 (M5) | Test Example 8 (M6) |
| Raw material name | Blending % | Blending % | Blending % | Blending % | Blending % | Blending % |
| Concentrated skim milk | 24.00 | 24.00 | 24.00 | 24.00 | 24.00 | 24.00 |
| Cream | 0.50 | 0.50 | 0.50 | 0.50 | 0.50 | 0.50 |
| (A) Casein hydrolysate | 0.50 | 0.50 | 0.50 | 0.50 | 0.50 | 0.50 |

EP 3 753 569 A1

(continued)

| Table 5: Drink yogurt (pre-fermented) | | | | | | % by mass |
|---|---|---|---|---|---|---|
| | Test Example 3 (M1) | Test Example 4 (M2) | Test Example 5 (M3) | Test Example 6 (M4) | Test Example 7 (M5) | Test Example 8 (M6) |
| Raw material name | Blending % | Blending % | Blending % | Blending % | Blending % | Blending % |
| (B) Indigestible dextrin | - | - | - | - | - | - |
| (C) Sucralose | - | 0.01 | - | 0.01 | - | 0.01 |
| (D) Rare sugar-containing syrup | - | - | 0.05 | 0.05 | - | 0.05 |
| (E) Lactulose-containing syrup | - | - | - | - | 2.00 | 2.00 |
| Starter | 0.01 | 0.01 | 0.01 | 0.01 | 0.01 | 0.01 |
| Water | 64.99 | 64.98 | 64.94 | 64.93 | 62.99 | 62.93 |
| Subtotal | 90 | 90 | 90 | 90 | 90 | 90 |
| | | | | | | |
| Pectin | 0.10 | 0.10 | 0.10 | 0.10 | 0.10 | 0.10 |
| Water | 9.90 | 9.90 | 9.90 | 9.90 | 9.90 | 9.90 |
| Subtotal | 10 | 10 | 10 | 10 | 10 | 10 |
| Total | 100.00 | 100.00 | 100.00 | 100.00 | 100.00 | 100.00 |

| Table 6: Drink yogurt (pre-fermented) | | | | | | % by mass |
|---|---|---|---|---|---|---|
| | Test Example 9(F1) | TestExample 10 (F2) | TestExample 11 (F3) | TestExample 12 (F4) | TestExample 13 (F5) | TestExample 14 (F6) |
| Raw material name | Blending % | Blending % | Blending % | Blending % | Blending % | Blending % |
| Concentrated skim milk | 24.00 | 24.00 | 24.00 | 24.00 | 24.00 | 24.00 |
| Cream | 0.50 | 0.50 | 0.50 | 0.50 | 0.50 | 0.50 |
| (A) Casein hydrolysate | - | - | - | - | - | - |
| (B) Indigestible dextrin | 5.50 | 5.50 | 5.50 | 5.50 | 5.50 | 5.50 |
| (C) Sucralose | - | 0.01 | - | 0.01 | - | 0.01 |
| (D) Rare sugar-containing syrup | - | - | 0.05 | 0.05 | - | 0.05 |
| (E) Lactulose-containing syrup | - | - | - | - | 2.00 | 2.00 |
| Starter | 0.01 | 0.01 | 0.01 | 0.01 | 0.01 | 0.01 |
| Water | 59.99 | 59.98 | 59.94 | 59.93 | 57.99 | 57.93 |
| Subtotal | 90 | 90 | 90 | 90 | 90 | 90 |

(continued)

| Table 6: Drink yogurt (pre-fermented) | | | | | | % by mass |
|---|---|---|---|---|---|---|
| | Test Example 9(F1) | TestExample 10 (F2) | TestExample 11 (F3) | TestExample 12 (F4) | TestExample 13 (F5) | TestExample 14 (F6) |
| Raw material name | Blending % | Blending % | Blending % | Blending % | Blending % | Blending % |
| | | | | | | |
| Pectin | 0.10 | 0.10 | 0.10 | 0.10 | 0.10 | 0.10 |
| Water | 9.90 | 9.90 | 9.90 | 9.90 | 9.90 | 9.90 |
| Subtotal | 10 | 10 | 10 | 10 | 10 | 10 |
| Total | 100.00 | 100.00 | 100.00 | 100.00 | 100.00 | 100.00 |

| Table 7: Drink yogurt (pre-fermented) | | | | | | % by mass |
|---|---|---|---|---|---|---|
| | TestExample 15 (MF1) | TestExample 16 (MF2) | TestExample 17 (MF3) | TestExample 18 (MF4) | TestExample 19 (MF5) | TestExample 20 (MF6) |
| Raw material name | Blending % | Blending % | Blending % | Blending % | Blending % | Blending % |
| Concentrated skim milk | 24.00 | 24.00 | 24.00 | 24.00 | 24.00 | 24.00 |
| Cream | 0.50 | 0.50 | 0.50 | 0.50 | 0.50 | 0.50 |
| (A) Casein hydrolysate | 0.50 | 0.50 | 0.50 | 0.50 | 0.50 | 0.50 |
| (B) Indigestible dextrin | 5.50 | 5.50 | 5.50 | 5.50 | 5.50 | 5.50 |
| (C) Sucralose | - | 0.01 | - | 0.01 | - | 0.01 |
| (D) Rare sugar-containing syrup | - | - | 0.05 | 0.05 | - | 0.05 |
| (E) Lactulose-containing-syrup | - | - | - | - | 2.00 | 2.00 |
| Starter | 0.01 | 0.01 | 0.01 | 0.01 | 0.01 | 0.01 |
| Water | 59.49 | 59.48 | 59.44 | 59.43 | 57.49 | 57.43 |
| Subtotal | 90 | 90 | 90 | 90 | 90 | 90 |
| | | | | | | |
| Pectin | 0.10 | 0.10 | 0.10 | 0.10 | 0.10 | 0.10 |
| Water | 9.90 | 9.90 | 9.90 | 9.90 | 9.90 | 9.90 |
| Subtotal | 10 | 10 | 10 | 10 | 10 | 10 |
| Total | 100.00 | 100.00 | 100.00 | 100.00 | 100.00 | 100.00 |

(1) Raw Material Preparation Step (I)

First, using a mixer, the raw materials shown in Table 4 to Table 7 were mixed together to prepare the fermented-milk raw material solution (containing no pectin) in each of Test Examples 1 to 20.

Subsequently, the fermented-milk raw material solution in each of Test Examples 1 to 20 was warmed and homogenized using a homogenizer (temperature, 85°C; pressure, 20 MPa). Each warmed and homogenized fermented-milk raw material solution was subjected to heat sterilization treatment (at 90°C for 10 minutes), and then cooled (42°C). Thus, a liquid milk preparation (containing no pectin) for the fermented milk in each of Test Examples 1 to 20 was prepared.

(2) Fermentation Step (II)

To the cooled liquid milk preparation in each of Test Examples 1 to 20, a lactic acid bacterial starter (mixed culture of *Streptococcus thermophilus* (*S. thermophilus)* and *Lactobacillus delbrueckii* subsp. *bulgaricus* (*L. delbrueckii* subsp. *bulgaricus*): YO-MIX[(R)]; manufactured by Danisco) was added, and then fermentation treatment (40°C, 7 hours) was carried out. Pectin was added to the resulting fermentation product as shown in Table 4 to Table 7, and the resulting mixture was cooled (5°C) and crushed, and then added to a container. Thus, the fermented milk in each of Test Examples 1 to 20 was prepared.

The measured concentration of each component in the fermented milk obtained in each of Test Examples 18 and 20 falls within the following range: (A) 0.3 to 0.8% by mass casein hydrolysate (0.0001 to 0.0005% by mass tripeptide MKP), (B) 4 to 8% by mass indigestible dextrin, (C) 0.005 to 0.02% by mass sucralose, (D) 0.015 to 0.1% by mass D-psicose and D-allose (psicose : allose (mass ratio) = 0.5 to 17 : 0.2 to 10), (E) 1 to 4% by mass lactulose.

Sensory evaluation (bitterness, starchy odor, unpleasant odor, fermented-milk flavor, and milk taste) was carried out for the resulting fermented milks of Test Examples 1 to 20.

Sensory Evaluation

The fermented milk of each Test Example was subjected to sensory evaluation by 10 panelists. The averages obtained are shown in Table 8 to Table 12.

Evaluation of Bitterness:

  4. Almost no bitterness

  3. Slight bitterness

  2. Moderate bitterness

  1. Strong bitterness

Evaluation of Starchy Odor:

  4. Almost no starchy odor
  3. Slight starchy odor
  2. Moderate starchy odor
  1. Strong starchy odor

Evaluation of Unpleasant Odor:

  4. Almost no unpleasant odor
  3. Slight unpleasant odor
  2. Moderate unpleasant odor
  1. Strong unpleasant odor

Evaluation of Fermented-Milk Flavor and Taste:

4. Strong fermented-milk flavor and taste
3. Moderate fermented-milk flavor and taste
2. Slight fermented-milk flavor and taste
1. Almost no fermented-milk flavor and taste

Evaluation of Milk Taste:

4. Strong milk taste
3. Moderate milk taste
2. Slight milk taste
1. Almost no milk taste

| Table 8: Bitterness evaluation | C: Control | M: (A) Casein digest | F: (B) Indigestible dextrin | MF: (A) Casein hydrolysate and (B) Indigestible dextrin |
|---|---|---|---|---|
| 1: No sugar | Test Example 1 (C1) | Test Example 3 (M1) | Test Example 9 (F1) | Test Example 15 (MF1) |
| Evaluation (bitterness) | 4 | 1 | 4 | 1 |
| 2: (C) Sucralose | | Test Example 4 (M2) | Test Example 10 (F2) | Test Example 16 (MF2) |
| Evaluation (bitterness) | | 3 | 4 | 3 |
| 3: (D) Rare-sugar syrup | | Test Example 5 (M3) | Test Example 11 (F3) | Test Example 17 (MF3) |
| Evaluation (bitterness) | | 1 | 4 | 2 |
| 4: (C) Sucralose + (D) Rare-sugar syrup | | Test Example 6 (M4) | Test Example 12 (F4) | Test Example 18 (MF4) |
| Evaluation (bitterness) | | 4 | 4 | 4 |
| 5: (E) Lactulose syrup | | Test Example 7 (M5) | Test Example 13 (F5) | Test Example 19 (MF5) |
| Evaluation (bitterness) | | 1 | 4 | 2 |
| 6: (C) Sucralose + (D) Rare-sugar syrup + (E) Lactulose syrup | Test Example 2(C2) | Test Example 8 (M6) | Test Example 14 (F6) | Test Example 20 (MF6) |
| Evaluation (bitterness) | 4 | 4 | 4 | 4 |

| Table 9: Starchy odor evaluation | C: Control | M: (A) Casein digest (MKP) | F: (B) Indigestible dextrin | MF: (A) Casein hydrolysate and (B) Indigestible dextrin |
|---|---|---|---|---|
| 1: No sugar | Test Example 1 (C1) | Test Example 3 (M1) | Test Example 9 (F1) | Test Example 15 (MF1) |
| Evaluation (starchy odor) | 4 | 4 | 1 | 1 |

(continued)

| Table 9: Starchy odor evaluation | C: Control | M: (A) Casein digest (MKP) | F: (B) Indigestible dextrin | MF: (A) Casein hydrolysate and (B) Indigestible dextrin |
|---|---|---|---|---|
| 2: (C) Sucralose | | Test Example 4 (M2) | Test Example 10 (F2) | Test Example 16 (MF2) |
| Evaluation (starchy odor) | | 4 | 2 | 2 |
| 3: (D) Rare-sugar syrup | | Test Example 5 (M3) | Test Example 11 (F3) | Test Example 17 (MF3) |
| Evaluation (starchy odor) | | 4 | 3 | 3 |
| 4: (C) Sucralose + (D) Rare-sugar syrup | | Test Example 6 (M4) | Test Example 12 (F4) | Test Example 18 (MF4) |
| Evaluation (starchy odor) | | 4 | 3 | 4 |
| 5: (E) Lactulose syrup | | Test Example 7 (M5) | Test Example 13 (F5) | Test Example 19 (MF5) |
| Evaluation (starchy odor) | | 4 | 2 | 2 |
| 6: (C) Sucralose + (D) Rare-sugar syrup + (E) Lactulose syrup | Test Example 2 (C2) | Test Example 8 (M6) syrup | Test Example 14 (F6) | Test Example 20 (MF6) |
| Evaluation (starchy odor) | 4 | 4 | 3 | 4 |

| Table 10: Unpleasant odor evaluation | C: Control | M: (A) Casein digest (MKP) | F: (B) Indigestible dextrin | MF: (A) Casein hydrolysate and (B) Indigestible dextrin |
|---|---|---|---|---|
| 1: No sugar | Test Example 1 (C1) | Test Example 3 (M1) | Test Example 9 (F1) | Test Example 15 (MF1) |
| Evaluation (unpleasant odor) | 4 | 2 | 2 | 1 |
| 2 : (C) Sucralose | | Test Example 4 (M2) | Test Example 10 (F2) | Test Example 16 (MF2) |
| Evaluation (unpleasant odor) | | 3 | 2 | 2 |
| 3: (D) Rare-sugar syrup | | Test Example 5 (M3) | Test Example 11 (F3) | Test Example 17 (MF3) |
| Evaluation (unpleasant odor) | | 2 | 2 | 2 |
| 4: (C) Sucralose + (D) Rare-sugar syrup | | Test Example 6 (M4) | Test Example 12 (F4) | Test Example 18 (MF4) |
| Evaluation (unpleasant odor) | | 4 | 3 | 3 |
| 5: (E) Lactulose syrup | | Test Example 7 (M5) | Test Example 13 (F5) | Test Example 19 (MF5) |
| Evaluation (unpleasant odor) | | 2 | 2 | 2 |
| 6: (C) Sucralose + (D) Rare-sugar syrup + (E) Lactulose syrup | Test Example 2 (C2) | Test Example 8 (M6) | Test Example 14 (F6) | Test Example 20 (MF6) |
| Evaluation (unpleasant odor) | 4 | 4 | 3 | 4 |

| Table 11: Evaluation of fermentative flavor and taste | C: Control | M: (A) Casein digest (MKP) | F: (B) Indigestible dextrin | MF: (A) Casein hydrolysate and (B) Indigestible dextrin |
|---|---|---|---|---|
| 1: No sugar | Test Example 1 (C1) | Test Example 3 (M1) | Test Example 9 (F1) | Test Example 15 (MF1) |
| Evaluation (fermentative flavor) | 4 | 3 | 2 | 2 |
| 2: (C) Sucralose | | Test Example 4 (M2) | Test Example 10 (F2) | Test Example 16 (MF2) |
| Evaluation (fermentative flavor) | | 2 | 1 | 1 |
| 3: (D) Rare-sugar syrup | | Test Example 5 (M3) | Test Example 11 (F3) | Test Example 17 (MF3) |
| Evaluation (fermentative flavor) | | 2 | 2 | 2 |
| 4: (C) Sucralose + (D) Rare-sugar syrup | | Test Example 6 (M4) | Test Example 12 (F4) | Test Example 18 (MF4) |
| Evaluation (fermentative flavor) | | 3 | 3 | 4 |
| 5: (E) Lactulose syrup | | Test Example 7 (M5) | Test Example 13 (F5) | Test Example 19 (MF5) |
| Evaluation (fermentative flavor) | | 3 | 3 | 3 |
| 6: (C) Sucralose + (D) Rare-sugar syrup + (E) Lactulose syrup | Test Example 2 (C2) | Test Example 8 (M6) | Test Example 14 (F6) | Test Example 20 (MF6) |
| Evaluation (fermentative flavor) | 4 | 4 | 3 | 4 |

| Table 12: Evaluation (milk taste) | C: Control | M: (A) Casein digest (MKP) | F: (B) Indigestible dextrin | MF: (A) Casein hydrolysate and (B) Indigestible dextrin |
|---|---|---|---|---|
| 1: No sugar | Test Example 1 (C1) | Test Example 3 (M1) | Test Example 9 (F1) | Test Example 15 (MF1) |
| Evaluation (milk taste) | 3 | 2 | 2 | 2 |
| 2: (C) Sucralose | | Test Example 4 (M2) | Test Example 10 (F2) | Test Example 16 (MF2) |
| Evaluation (milk taste) | | 2 | 2 | 2 |
| 3: (D) Rare-sugar syrup | | Test Example 5 (M3) | Test Example 11 (F3) | Test Example 17 (MF3) |
| Evaluation (milk taste) | | 2 | 2 | 2 |
| 4: (C) Sucralose + (D) Rare-sugar syrup | | Test Example 6 (M4) | Test Example 12 (F4) | Test Example 18 (MF4) |
| Evaluation (milk taste) | | 3 | 2 | 2 |

(continued)

| Table 12: Evaluation (milk taste) | C: Control | M: (A) Casein digest (MKP) | F: (B) Indigestible dextrin | MF: (A) Casein hydrolysate and (B) Indigestible dextrin |
|---|---|---|---|---|
| 5: (E) Lactulose syrup | | Test Example 7 (M5) | Test Example 13 (F5) | Test Example 19 (MF5) |
| Evaluation (milk taste) | | 3 | 3 | 4 |
| 6: (C) Sucralose + (D) Rare-sugar syrup + (E) Lactulose syrup | Test Example 2 (C2) | Test Example 8 (M6) | Test Example 14 (F6) | Test Example 20 (MF6) |
| Evaluation (milk taste) | 4 | 4 | 3 | 4 |

These results indicate that, in cases where sucralose alone is used, a masking effect on the bitterness and the starchy odor caused by the milk protein hydrolysate and/or the indigestible dextrin can be produced, but the fermented-milk taste is deteriorated.

In cases where a rare sugar-containing syrup alone is used, a masking effect on the starchy odor caused by the indigestible dextrin can be produced, but there is also a masking effect on the fermentative flavor and taste of the fermented milk, so that the taste of the fermented milk is deteriorated. In cases where a lactulose-containing syrup alone is used, the masking effect on the unpleasant odor caused by the milk protein hydrolysate and/or the indigestible dextrin is small. Thus, when the fermented milk contains a milk protein hydrolysate and/or an indigestible dextrin, and the above effects are to be expected, the use of sucralose, a rare sugar-containing syrup, or a lactulose-containing syrup alone results in deterioration of the taste of the fermented milk.

However, in cases where sucralose + rare sugar-containing syrup are used in combination, a higher masking effect on the bitterness, starchy odor, and unpleasant odor can be produced for a fermented milk containing a milk protein hydrolysate and/or an indigestible dextrin, compared to cases where they are individually used. Furthermore, the combined use allows maintenance of the fermentative flavor, and does not cause deterioration of the taste of the fermented milk.

In cases where a lactulose-containing syrup is further used in combination with sucralose + rare sugar-containing syrup, a further milk taste and fermentative flavor can be effectively given to a fermented milk containing a milk protein hydrolysate and/or an indigestible dextrin, resulting in improvement or enhancement of the taste of the fermented milk.

Further, as shown by Test Examples 21 to 24, also in the cases of yogurts in a wide range of forms, combined use of sucralose + rare sugar-containing syrup, or additional use of a lactulose-containing syrup in combination therewith, produces a high masking effect on the bitterness, starchy odor, and unpleasant odor for a fermented milk containing a milk protein hydrolysate and/or an indigestible dextrin, while allowing maintenance of the fermentative flavor, and without deteriorating the taste of the fermented milk, so that the taste of the fermented milk can be improved.

Test Examples 21 and 22: Drink Yogurt (Pre-fermentation)

(1) Raw Material Preparation Step (I)

First, using a mixer, the raw materials shown in Table 13 were mixed together to prepare the fermented-milk raw material solution (containing no pectin) in each of Test Examples 21 and 22.

Subsequently, the fermented-milk raw material solution in each of Test Examples 21 and 22 was warmed and homogenized using a homogenizer (temperature, 85°C; pressure, 20 MPa). Each warmed and homogenized fermented-milk raw material solution was subjected to heat sterilization treatment (90°C, 10 minutes), and then cooled (42°C). Thus, a liquid milk preparation for the fermented milk in each of Test Examples 21 and 22 was prepared.

(2) Fermentation Step (II)

To the cooled liquid milk preparation in each of Test Examples 21 and 22, a lactic acid bacterial starter (mixed culture of *Streptococcus thermophilus* (*S. thermophilus*) and *Lactobacillus delbrueckii* subsp. *bulgaricus* (*L. delbrueckii* subsp. *bulgaricus*): YO-MIX(R); manufactured by Danisco) was added, and then fermentation treatment (40°C, 7 hours) was carried out. The resulting fermentation product was cooled (5°C) and crushed, and then filled into a container. Thus, the fermented milk in each of Test Examples 21 and 22 was prepared.

Sensory evaluation (bitterness, starchy odor, unpleasant odor, fermented-milk flavor, and milk taste) was carried out for the resulting fermented milks of Test Examples 21 and 22.

In the fermented milk of each of Test Examples 21 and 22, the bitterness, starchy odor, and unpleasant odor were masked, and a pleasant fermented-milk flavor was maintained. Thus, the taste of the fermented milk obtained was good. The fermented milk of Test Example 22 showed improvement of the unpleasant odor, and had a better milk taste, compared to the fermented milk of Test Example 21.

| Table 13: Drink yogurt (pre-fermented) | | % by mass |
|---|---|---|
| | Test Example 21 | Test Example 22 |
| Raw material name | Blending % | Blending % |
| Concentrated skim milk | 24.00 | 24.00 |
| Cream | 0.50 | 0.50 |
| (A) Casein hydrolysate | 0.50 | 0.50 |
| (B) Indigestible dextrin | 5.50 | 5.50 |
| (C) Sucralose | 0.01 | 0.01 |
| (D) Rare sugar-containing syrup | 0.05 | 0.05 |
| (E) Lactulose-containing syrup | - | 2.00 |
| Flavoring agent | 0.20 | 0.20 |
| Starter | 0.01 | 0.01 |
| Water | 69.23 | 67.23 |
| Total | 100 | 100 |

Test Examples 23 and 24: Fermented Milk: Solid-Form Yogurt

The raw materials used for production of fermented milk: solid-form yogurts (stationary yogurts: post-fermented type) are shown in Table 14.

(1) Raw Material Preparation Step (I)

First, using a mixer, the raw materials shown in Table 14 were mixed together to prepare the fermented-milk raw material solution in each of Test Examples 23 and 24.

Subsequently, the fermented-milk raw material solution in each of Test Examples 23 and 24 was warmed and homogenized using a homogenizer (temperature, 85°C; pressure, 20 MPa). Each warmed and homogenized fermented-milk raw material solution was subjected to heat sterilization treatment (90°C, 10 minutes), and then cooled (42°C). Thus, a liquid milk preparation for the fermented milk in each of Test Examples 21 and 22 was prepared.

(2) Fermentation Step (II)

To the cooled liquid milk preparation in each of Test Examples 23 and 24, a lactic acid bacterial starter (mixed culture of *Streptococcus thermophilus* (*S. thermophilus*) and *Lactobacillus delbrueckii* subsp. *bulgaricus* (*L. delbrueckii* subsp. *bulgaricus*): YO-MIX[(R)] U; manufactured by Danisco) was added, and the resulting mixture was filled into a container. Thereafter, the milk preparation in the container was subjected to fermentation treatment (40°C, 7 hours), and then refrigerated (10°C or lower). Thus, the fermented milk (stationary, coagulated yogurt) in each of Test Examples 23 and 24 was prepared.

Sensory evaluation (bitterness, starchy odor, unpleasant odor, fermented-milk flavor, and milk taste) was carried out for the resulting fermented milks of Test Examples 23 and 24.

The fermented milk of Test Example 23 showed excellent results in terms of the bitterness, starchy odor, unpleasant odor, and fermented-milk flavor. The fermented milk of Test Example 24 showed excellent results in terms of the bitterness, starchy odor, unpleasant odor, fermented-milk flavor, and milk taste. Regarding the unpleasant odor and the milk taste, the fermented milk of Test Example 24 showed better results than the fermented milk of Test Example 23.

| Table 14: Coagulated yogurt (post-fermented) | | % by mass |
|---|---|---|
| | Test Example 23 | Test Example 24 |
| Raw material name | Blending % | Blending % |
| Concentrated skim milk | 24.00 | 24.00 |
| Cream | 0.50 | 0.50 |
| Casein hydrolysate | 0.50 | 0.50 |
| Indigestible dextrin | 5.50 | 5.50 |
| Sucralose | 0.01 | 0.01 |
| Rare sugar-containing syrup | 0.05 | 0.05 |
| Lactulose-containing syrup | - | 2.00 |
| Agar | 0.10 | 0.10 |
| Flavoring agent | 0.20 | 0.20 |
| Starter | 0.01 | 0.01 |
| Water | 69.13 | 67.13 |
| Total | 100 | 100 |

**Claims**

1.  A composition for lowering blood pressure and/or reducing neutral fat, comprising a casein hydrolysate and an indigestible dextrin.

2. The composition according to claim 1, wherein the casein hydrolysate comprises a peptide composed of Met-Lys-Pro.

3. The composition according to claim 1 or 2, comprising the indigestible dextrin and the casein hydrolysate at a mass ratio of 10:0.001 to 1:1.

4. The composition according to any one of claims 1 to 3, wherein the casein hydrolysate comprises a peptide composed of Met-Lys-Pro, and the indigestible dextrin is present at 5,000 to 100,000 parts by mass with respect to 1 part by mass of the peptide.

5. The composition according to any one of claims 1 to 4, wherein the composition is a pharmaceutical composition or a food or beverage composition.

6. The composition according to claim 5, which is used for lowering blood pressure and/or for reducing neutral fat.

7. The composition according to claim 5 or 6, which is used for the prevention or treatment of hypertension and/or dyslipidemia.

8. The composition according to any one of claims 1 to 6, in the form of a unit package.

9. The composition according to any one of claims 1 to 5, wherein the composition is a fermented food or beverage.

10. The composition according to claim 9, wherein the fermented food or beverage comprises sucralose, and one or more monosaccharides and/or rare sugars selected from the group consisting of fructose, psicose, and allose.

11. The composition according to claim 10, further comprising lactulose.

12. A method of producing a fermented milk comprising a casein hydrolysate and an indigestible dextrin, and further comprising sucralose, and one or more monosaccharides and/or rare sugars selected from the group consisting of fructose, psicose, and allose.

13. The method of producing a fermented milk according to claim 12, wherein the casein hydrolysate comprises a peptide composed of Met-Lys-Pro.

14. The method of producing a fermented milk according to claim 12 or 13, wherein the fermented milk further comprises lactulose.

# EP 3 753 569 A1

**INTERNATIONAL SEARCH REPORT**

International application No.

PCT/JP2019/005301

**A. CLASSIFICATION OF SUBJECT MATTER**

Int.Cl. A61K38/17(2006.01)i, A23L2/00(2006.01)i, A23L33/18(2016.01)i, A23L33/21(2016.01)i, A61K31/718(2006.01)i, A61P3/06(2006.01)i, A61P9/12(2006.01)i, C07K5/062(2006.01)i

According to International Patent Classification (IPC) or to both national classification and IPC

**B. FIELDS SEARCHED**

Minimum documentation searched (classification system followed by classification symbols)

Int.Cl. A61K38/17, A23L2/00, A23L33/18, A23L33/21, A61K31/718, A61P3/06, A61P9/12, C07K5/062

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

JSTPlus/JMEDPlus/JST7580 (JDreamIII), CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 2017-31105 A (MORINAGA MILK INDUSTRY CO., LTD.) 09 February 2017, (claims, paragraphs [0068], [0072], [0087]) (Family: none) | 12-14 |
| Y | JP 2000-135055 A (SANEI GEN FFI INC.) 16 May 2000 (claims, example 1) & US 2007/0212460 A1 (claims, example 1) & EP 1210880 A1 & AU 6366399 A | 10-14 |

☒ Further documents are listed in the continuation of Box C. ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 29 March 2019 (29.03.2019) | 09 April 2019 (09.04.2019) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

44

**INTERNATIONAL SEARCH REPORT**

| International application No. |
| --- |
| PCT/JP2019/005301 |

C (Continuation).  DOCUMENTS CONSIDERED TO BE RELEVANT

| Category* | Citation of document, with indication, where appropriate, of the relevant passages | Relevant to claim No. |
| --- | --- | --- |
| Y | JP 4540231 B1 (SANEI GEN FFI INC.) 08 September 2010 (pp. 18-20, 33, example II-1-22, pp. 70-71) & US 7229658 B1 (pp. 22-23, 42-44, example (II-1-2(2)) & EP 1210880 A1 & AU 6366399 A | 10-14 |
| Y | JP 2009-167172 A (NUTRI CO., LTD.) 30 July 2009 (claims, adjustment examples, examples, paragraph [0019]) (Family: none) | 10-14 |
| Y | JP 5-176713 A (MORINAGA MILK INDUSTRY CO., LTD.) 20 July 1993 (test example 3, table 4, test group 8-10, paragraph [0047], example 3) (Family: none) | 1-11 |
| Y | JP 2015-36376 A (MORINAGA MILK INDUSTRY CO., LTD.) 23 February 2015 (claims, paragraphs [0003], [0006], [0008], [0038]-[0044], production example 1, paragraphs [0052], [0054]-[0056], table 1, fig. 1) (Family: none) | 1-11 |
| Y | JP 2016-69343 A (MORINAGA MILK INDUSTRY CO., LTD.) 09 May 2016 (paragraphs [0062]-[0065]) (Family: none) | 1-11 |
| Y | 金子 清久 他, 難消化性デキストリン含有茶飲料摂取による食後血中中性脂肪上昇抑制 －無作為化プラセボ対照二重盲検クロスオーバー試験－, Jpn Pharmacol Ther., 2016, vol. 44, no. 2, pp. 263-270 (ISSN:0386-3603) (pp. 267-269, II Results, conclusion, abstract, etc.), non-official translation (KANEKO, Kiyohisa et al., "Suppression of increase in postprandial neutral fat in blood by ingestion of indigestible dextrin-containing tea beverage – A randomized, placebo-controlled, double-blind, cross-over study –") | 1-11 |
| Y | 岸本 由香, 松谷化学の機能性素材（難消化性デキストリン、希少糖含有シロップ）を活用した機能性表示食品の開発, 月刊フードケミカル, 2016, vol. 373, pp. 77-80 (ISSN:0911-2286) (p. 78 4) Foods with function claims, table 1), non-official translation (KISHIMOTO, Yuka, "Development of foods with function claims, using Matsutani Chemical's functional ingredients (indigestible dextrin, rare sugar syrup)", A Technical Journal on Food Chemistry & Chemicals) | 1-11 |

Form PCT/ISA/210 (continuation of second sheet) (January 2015)

**INTERNATIONAL SEARCH REPORT**

International application No.

PCT/JP2019/005301

C (Continuation).    DOCUMENTS CONSIDERED TO BE RELEVANT

| Category* | Citation of document, with indication, where appropriate, of the relevant passages | Relevant to claim No. |
|---|---|---|
| Y | 越智 大介 他、 カゼイン由来トリペプチド Met-Lys-Pro の正常高値血圧者および 1 度高血圧者に対する血圧降下作用 －ランダム化二重盲検プラセボ対照平行群間比較試験－ Jpn Pharmacol Ther., 2017, vol. 45, no. 10, pp. 1637-1648 (ISSN:0386-3603) (p. 1638, introduction, p. 1642, left column, result, 2 Blood pressure, conclusion, abstract), (OCHI, Daisuke et al., "Antihypertensive effect of the casein-derived peptide Met-Lys-Pro in individuals with high-normal blood pressure or grade 1 hypertension - A randomized, double-blind, placebo-controlled, parallel-group trial -") | 1-11 |
| Y | 社団法人菓子総合技術センター，難消化性デキストリン，農林水産省食品流通局委託事業 飲食料品機能性素材有効利用技術シリーズ No.9, 1991, pp. 1-25, (p. 7 6) Hypotensive action), non-official translation (Japan Confectionery Research Center, "Indigestible dextrin", Consignment of the Ministry of Agriculture, Forestry and Fisheries Food and Marketing Bureau, Technology series No. 9 for the effective utilization of dietary functional materials) | 1-11 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2001252064 A
- WO 2003044044 A
- WO 2013125622 A
- JP 2016069343 A
- JP 4091765 A

- JP 2017031105 A
- JP 2017184682 A
- WO 2010113785 A
- JP 3169888 A
- JP 6228179 A

### Non-patent literature cited in the description

- The Society of Polymer Science. Basic High Polymer Science. Tokyo Kagaku Dojin Co. Ltd, 1978, 116-119
- Analytical Methods for Nutrients on the Standards for Nutrition Labeling. *EISHIN No.13,* 26 April 1999
- Indigestible Fractions of Starch Hydrolysates and Their Determination Method. Kazuhiro Okuma and Isao Matsuda. *J. Appl. Glycosci.,* 2002, vol. 49 (4), 479-485

- **KAZUHIRO OHKUMA ; ISAO MATSUDA ; YASUO KATTA ; YOSHIO HANNO.** Pyrolysis of Starch and Its Digestibility by Enzymes -Characterization of Indigestible Dextrin-. *Denpun kagaku,* 1990, vol. 37 (2), 107-114
- **KAZUHIRO OHKUMA ; ISAO MATSUDA.** Production of Indigestible Dextrin from Pyrodextrin. *J. Appl. Glycosci.,* 2003, vol. 50 (3), 389-394
- *Japan Ministry of Health, Labor and Welfare, Ministerial Ordinance No. 86,* 30 April 2003
- *Food Labeling Act,* 2013
- New Food Processing Lecture: Milk and Its Processing (saishin shokuhinkakokoza: nyu to sono kako). Kenpakusha Co., Ltd, 1987, 282

SEQUENCE LISTING

<110>   MORINAGA MILK INDUSTRY CO.,LTD.

<120>   THE COMPOSITION OF HYPOTENSION and/or NEUTRAL FAT REDUCER

<130>   FPCT764

<150>   JP2018-26410
<151>   2018-02-16

<150>   JP2018-26420
<151>   2018-02-16

<160>   1

<170>   PatentIn version 3.5

<210>   1
<211>   3
<212>   PRT
<213>   Artificial Sequence

<400>   1

Met Lys Pro
1